# EUROPÄISCHE PATENTSCHRIFT

(11) **EP 3 024 487 B1**
(45) Veröffentlichungstag und Bekanntmachung des Hinweises auf die Patenterteilung: **06.11.2019**
(21) Anmeldenummer: 14744820.3
(22) Anmeldetag: 25.07.2014
(51) Int. Cl.: A61K 41/00, A61L 2/00, A61K 39/145

(54) **VERFAHREN ZUR INAKTIVIERUNG VON VIREN UNTER VERWENDUNG VON ELEKTRONENSTRAHLEN**
METHOD FOR INACTIVATING VIRUSES USING ELECTRON BEAMS
PROCÉDÉ PERMETTANT L'INACTIVATION DE VIRUS AU MOYEN DE FAISCEAUX ÉLECTRONIQUES

(30) Priorität: 26.07.2013 DE 102013012455
(43) Veröffentlichungstag der Anmeldung: 01.06.2016
(73) Patentinhaber: Fraunhofer Gesellschaft zur Förderung der angewandten Forschung E.V., 80686 München (DE)
(72) Erfinder: ULBERT, Sebastian, 04277 Leipzig (DE); WETZEL, Christiane, 01217 Dresden (DE)
(74) Vertreter: Lahrtz, Fritz
(86) Internationale Anmeldenummer: PCT/EP2014/066039
(87) Internationale Veröffentlichungsnummer: WO 2015/011265

(56) Entgegenhaltungen:
- CA-A- 631 016
- GB-A- 899 011
- V. BRAHMAKSHATRIYA ET AL: "Preliminary study for evaluation of avian influenza virus inactivation in contaminated poultry products using electron beam irradiation", AVIAN PATHOLOGY, Bd. 38, Nr. 3, 1. Juni 2009 (2009-06-01), Seiten 245-250, XP055025915, ISSN: 0307-9457, DOI: 10.1080/03079450902912150
- PREUSS T ET AL: "Comparison of two different methods for inactivation of viruses in serum", CLINICAL AND DIAGNOSTIC LABORATORY IMMUNOLOGY 199709 US, vol. 4, no. 5, September 1997 (1997-09), pages 504-508, XP55545680, ISSN: 1071-412X
- STAUFFER FAUSTO ET AL: "Advances in the development of inactivated virus vaccines.", RECENT PATENTS ON ANTI-INFECTIVE DRUG DISCOVERY NOV 2006, vol. 1, no. 3, November 2006 (2006-11), pages 291-296, XP55545601, ISSN: 1574-891X
- KUMAR ET AL: "Utilization of 10 MeV RF electron linear accelerator for research and industrial applications", INDIAN JOURNAL OF PURE & APPLIED PHYSICS, 1 November 2012 (2012-11-01), pages 802-804,

## Beschreibung

Die Erfindung betrifft ein Verfahren zur Inaktivierung von Viren, dadurch gekennzeichnet, dass eine immunogene Zusammensetzung oder Vakzine enthaltend mindestens ein Virus mit Elektronenstrahlen bestrahlt wird, wobei die immunogene Zusammensetzung oder Vakzine enthaltend mindestens ein Virus (i) flüssig ist, insbesondere eine Suspension ist, und (ii) mindestens ein virales Immunogen enthält.

Durch den Einsatz von Impfstoffen können viele Infektionskrankheiten in der Human- und Veterinärmedizin erfolgreich bekämpft werden. Trotzdem gibt es immer noch einen großen Bedarf an Impfstoff-Technologien, die effektiv und langandauernd vor einer Infektion schützen, dabei aber für das geimpfte Individuum ohne Risiken sind. Dies wird bei der Herstellung von sogenannten Tot-Impfstoffen deutlich: für die Inaktivierung von Viren werden toxische Chemikalien wie Formaldehyd verwendet, welche dann durch aufwendige Verfahren wieder aus dem Impfstoff entfernt werden müssen. In der Veterinärmedizin machen Formaldehyd-inaktivierte Impfstoffe den Großteil aller Vakzine aus, in der Humanmedizin werden sie z. B. gegen FSME, Grippe, Kinderlähmung oder Hepatitis A eingesetzt. Die Verwendung von Formaldehyd führt zu einer chemischen Veränderung (Kreuz-Vernetzung) der viralen Antigene. Dies resultiert wiederum in einer abgeschwächten Wirksamkeit des Impfstoffs, was durch erhöhte Mengen an infektiösem Ausgangsmaterial und Wirkungs-Verstärker (Adjuvantien) ausgeglichen werden muss. Eine Technik, welche diese Probleme umgeht, ist ein klares unmet need der Impfstoff-Industrie.

Studien haben gezeigt, dass durch Formaldehyd die für eine erfolgreiche Impfung wichtigen Antigene zu 30%-80% zerstört werden (Amanna et al., Nature Medicine, 18, 2012). Diese Probleme sind der Impfstoff-Industrie bekannt und man sucht nach Alternativen. So wird mit UV-Strahlen, erhöhten Temperaturen, Gamma-Strahlen oder Peroxiden experimentiert. Bisher hat jedoch noch keine dieser Techniken die experimentelle Labor-Phase verlassen. Unterschiedliche Verfahren zur Inaktivierung von Viren zur Herstellung eines Impfstoffes werden beispielsweise in Stauffer et al. beschrieben (Recent Patents on Anti-Infective Drug Discovery, 2006, 1, 291-296).

Weiterhin ist beschrieben worden, dass Salmonellen Populationen unter Einsatz von hochenergetischen Elektronenstrahlen reduziert werden können (US 8,173,139B1). Es wird dort jedoch nicht beschrieben, inwieweit die Struktur von Antigenen beeinträchtigt wird. Salmonellen sind darüber hinaus Lebewesen mit einem eigenen Metabolismus. Eine Anwendbarkeit von Elektronenstrahlen auf Viren, welche außerhalb ihrer Wirtszellen über keinen eigenen Stoffwechsel verfügen, war daher nicht anzunehmen.

In der vorliegenden Erfindung wurde nun überraschend gefunden, dass Viren mit Elektronenstrahlen inaktiviert werden konnten, ohne dass virale Strukturproteine dabei zerstört wurden. Daher eignet sich die Bestrahlung mit Elektronenstrahlen überraschenderweise insbesondere zur Herstellung inaktivierter viraler Ganzpartikelimpfstoffe.

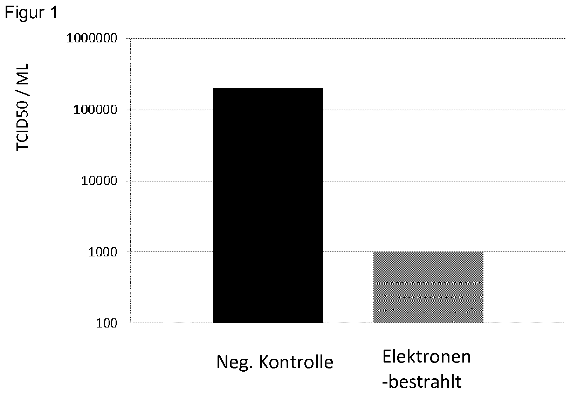

Die Inaktivierung der Viren erfolgt vermutlich durch die Zerstörung der Nukleinsäure, wobei die Virus- und insbesondere die Antigen-Struktur nicht oder kaum beschädigt wird. Die Ergebnisse am Beispiel des schweine-pathogenen PRRSV Virus sind in den Beispielen 1 und 2 sowie den Figuren 1 bis 3 dargestellt. Dabei wurde eine Flüssigkeit, nämlich ein Suspension dieser Viren in gepufferter wässriger Lösung mit Elektronenstrahlen behandelt. Diese Suspension ist immunogen, und eignet sich als Impfstoff gegen PRRSV für Schweine.

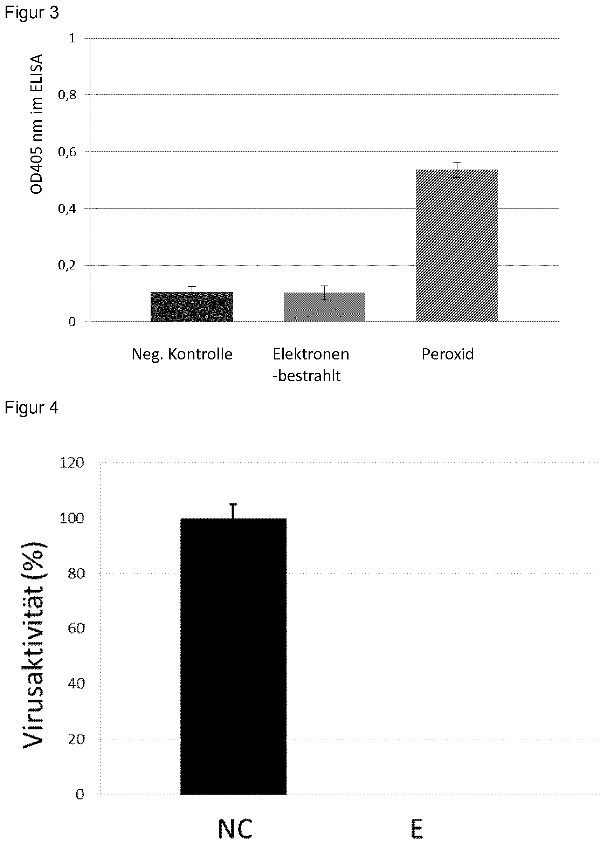

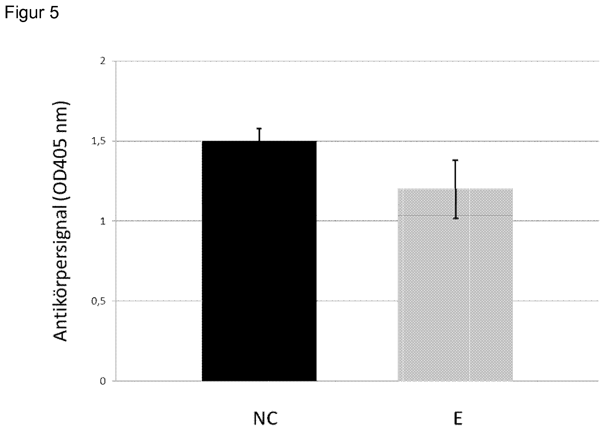

Die Ergebnisse mit dem Influenza A Virus sind im Beispiel 3 und den Figuren 4 und 5 dargestellt. Dabei wurde eine Flüssigkeit, nämlich ein Suspension dieser Viren in gepufferter wässriger Lösung mit Elektronenstrahlen behandelt. Diese Suspension ist immunogen, und eignet sich als Impfstoff gegen Influenza A.

Offenbart wird ein Verfahren zur Inaktivierung von Viren, dadurch gekennzeichnet, dass eine immunogene Zusammensetzung oder Vakzine enthaltend mindestens ein Virus mit Elektronenstrahlen bestrahlt wird, wobei die immunogene Zusammensetzung oder Vakzine enthaltend mindestens ein Virus (i) flüssig ist, insbesondere eine Suspension ist, und (ii) mindestens ein virales Immunogen enthält.

Insbesondere betrifft die vorliegende Erfindung ein Verfahren zur Inaktivierung von Viren, dadurch gekennzeichnet, dass eine immunogene Zusammensetzung oder Vakzine enthaltend mindestens ein Virus mit Elektronenstrahlen bestrahlt wird,
wobei die immunogene Zusammensetzung oder Vakzine enthaltend mindestens ein Virus mit einer Elektronenstrahldosis im Bereich von 15 bis 110 kGy bestrahlt wird und die Bestrahlungszeit im Bereich von zwischen 1 Sekunde und 100 Sekunden ist, und die Elektronenstrahlen mit einer Beschleunigungsenergie von zwischen 150 keV und 700 keV beschleunigt werden, und die immunogene Zusammensetzung oder Vakzine enthaltend mindestens ein Virus (i) flüssig ist, insbesondere eine Suspension ist, und (ii) mindestens ein virales Immunogen enthält.

In einer bevorzugten Ausführungsform ist das mindestens eine Virus vor der Bestrahlung aktiv und/oder die immunogene Zusammensetzung oder Vakzine enthält aktive Viren. Insbesondere beträgt die Konzentration an aktiven Viren vor der Bestrahlung in der flüssigen Zusammensetzung oder Vakzine, gemessen als TCID50-Wert (50% infektiöse Dosis in Gewebekultur) pro ml Flüssigkeit, mindestens 10⁴, 10⁵, oder 10⁶ pro ml.

Offenbart wird ein Verfahren zur Inaktivierung von Viren, dadurch gekennzeichnet, dass eine Zusammensetzung enthaltend mindestens ein Virus mit Elektronenstrahlen bestrahlt wird, wobei die Zusammensetzung enthaltend mindestens ein Virus (i) flüssig ist, insbesondere eine Suspension ist, und/oder (ii) eine immunogene Zusammensetzung ist.

Das Verfahren kann auf beliebige Viren, insbesondere behüllte oder unbehüllte Viren, angewendet werden.

Es ist in einer bevorzugten Ausführungsform für behüllte Viren geeignet, da Hüllproteine geeignete Antigene für eine Impfreaktion darstellen. Die Antigenstruktur bleibt mit dem erfindungsgemäßen Verfahren erhalten, während die Viren selbst inaktiviert werden (siehe Figuren 1, 2 und 3). Es kann daher mit dem erfindungsgemäßen Verfahren überraschenderweise insbesondere ein inaktivierter viraler Ganzpartikelimpfstoff für behüllte Viren hergestellt werden.

In einer anderen bevorzugten Ausführungsform kann ein inaktivierter viraler Ganzpartikelimpfstoff für unbehüllte Viren hergestellt werden. Ein geeignetes Antigen für eine Impfreaktion bei unbehüllten Viren ist insbesondere ein Capsid Protein des Virus.

Folgende Viren können beispielsweise erfindungsgemäß bestrahlt werden:
Behüllte Viren, beispielsweise:
   Doppelsträngige DNA-Viren, beispielsweise:
   Pockenvirus (verursacht Pocken);
   Herpesviren (z.B. Herpes simplex (HSV) (verursacht Herpes labialis oder genitalis); Varizella Zoster Virus (VZV) (verursacht Windpocken und Gürtelrose)
   Epstein-Barr-Virus (EBV) (verursacht Pfeiffer'sches Drüsenfieber) Zytomegalievirus (CMV) (verursacht Zytomegalie) Humanes Herpes Virus 6 7 (verursacht Drei-Tage-Fieber) Humanes Herpes Virus 8 (HHV 8) (verursacht Kaposi-Sarkom)
   Hepadnaviren, beispielsweise:
   Hepatitis B Virus (verursacht Hepatitis B) (+)-Strang-RNA-Viren, beispielsweise:
   Flaviviren, beispielsweise:
      Hepatitis C Virus (verursacht Hepatitis C)
   Togaviren, beispielsweise:
      Röteln-Virus (verursacht Röteln)
   Coronaviren (verursacht Magen-Darm-Entzündungen, SARS) (-)-Strang-RNA-Viren, beispielsweise::
   Orthomyxoviren, beispielsweise:
      Influenzaviren A, B, oder C (verursacht Influenza)
   Paramyxoviren, beispielsweise:
      Parainfluenzaviren (verursacht Parainfluenza)
      Masernvirus (verursacht Masern)
      Mumpsvirus (verursacht Mumps)
      Respiratory Sincytical Virus (RSV)
      Pneumoviridae, beispielsweise Gattungen: Pneumovirus, Metapneumovirus
   Rhabdoviren, beispielsweise:
      Tollwut-Virus (verursacht Tollwut)
   Retroviren, beispielsweise:
   Humanes Immunodefizienz Virus (verursacht AIDS)
   HTLV (verursacht Leukämie)
Unbehüllte Viren, beispielsweise:
   Doppelsträngige DNA-Viren, beispielsweise:
      Adenoviren (verursacht Schnupfen Erkältungen)
      Papoaviren (verursacht Warzen)
   Einzelsträngige DNA-Viren, beispielsweise:
      Parvoviren, beispielsweise:
      Parvovirus B19 (verursacht Ringelröteln)
   Doppelsträngige RNA-Viren, beispielsweise:
      Rotaviren (Durchfall)
   (+)-Strang-RNA-Viren, beispielsweise:
      Picornaviren, beispielsweise:
         Poliovirus (verursacht Kinderlähmung)
         Coxsachie - Viren
         Echo - Viren
         Hepatitis A Virus (verursacht Hepatitis A)
         Rhinoviren (verursacht Schnupfen Erkältungen)
      Calciviren (verursacht Durchfall)

In einer bevorzugten Ausführungsform ist der mindestens eine Virus daher ausgewählt aus:
(i) einem behüllten Virus oder unbehüllten Virus, insbesondere einem behüllten Virus, und/oder
(ii) einem dsDNA Virus, dsRNA Virus, ssRNA Virus, oder ssDNA Virus, und/oder
(iii) einem humanpathogenen und/oder tierpathogenen Virus.

In einer bevorzugten Ausführungsform ist das Tier ein Säugetier, insbesondere ausgewählt aus Schweinen, Kühen, Pferden, Hunden, Katzen und Schafen.

In einer stärker bevorzugten Ausführungsform ist das mindestens eine Virus ausgewählt ist aus einem humanpathogenen und/oder tierpathogenen behüllten dsRNA Virus, behüllten ss(-)RNA Virus, oder behüllten ss(+)RNA Virus.

Es wurde in den Beispielen gezeigt, dass der PRRS-Virus (*porcine respiratory and reproductive failure syndrome virus),* ein einzelsträngiges, positiv-Strang-RNA Virus aus der Familie *Arteriviridae,* das Schweine befällt, mit dem erfindungsgemäßen Verfahren so inaktiviert wurde, dass die Virusstruktur und Antigenstruktur weitgehend erhalten blieb. Somit konnte ein inaktivierter viraler Ganzpartikelimpfstoff gegen das PRRS-Virus hergestellt werden.

In einer noch stärker bevorzugten Ausführungsform ist das mindestens eine Virus ausgewählt aus einem humanpathogenen und/oder tierpathogenen ss(+)RNA-Virus, ganz besonders bevorzugt aus einem Virus der *Arteriviridae* Familie, noch mehr bevorzugt ist das mindestens eine Virus ein *porcine reproductive and respiratory syndrome virus* (PRRS Virus).

In einer weiteren bevorzugten Ausführungsform ist das mindestens eine Virus ausgewählt aus einem Echo-Virus, HI-Virus, Rotavirus, Pseudorabies Virus, Parvovirus, Schweine Parvovirus, H5N1 Virus, H1N1 Virus, Epstein-Barr-Virus, Mumps-Virus Influenza A und B Virus, dem FSME Virus, dem IPV Virus und dem Hepatitis A Virus.

In einer weiteren bevorzugten Ausführungsform ist das mindestens eine Virus ausgewählt aus einem tierpathogenen Virus, dem Influenza A und B Virus, dem FSME Virus, dem IPV Virus und dem Hepatitis A Virus. Besonders bevorzugt sind das Influenza A und B Virus.

Mit dem erfindungsgemäßen Verfahren ist es möglich, dass eine immunogene Zusammensetzung oder Vakzine enthaltend einen (1) Virus bestrahlt wird, wie in den Beispielen mit PRRSV dargestellt. Es ist jedoch auch möglich, dass die immunogene Zusammensetzung oder Vakzine 2, 3, 4, 5, 6, 7, 8, 9, 10 oder mehr unterschiedliche Viren enthält. Dies kann beispielsweise zur Herstellung von Kombinationsimpfstoffen hilfreich sein. Diese zwei oder mehr unterschiedliche Viren können Varianten der gleichen Virusspezies sein, oder Viren unterschiedlicher Spezies, Familien oder Gattungen. Daher enthält in einer bevorzugten Ausführungsform die immunogene Zusammensetzung oder Vakzine (i) einen Virus oder (ii) zwei oder mehr unterschiedliche Viren. Es ist aber auch möglich einen Kombinationsimpfstoff herzustellen, indem zwei oder mehr Viren einzeln bestrahlt werden, und erst nach der Bestrahlung kombiniert werden.

In den Beispielen wurde eine Suspension von PRRSV Viren mit Elektronen bestrahlt, die auf weniger als 300 KeV beschleunigt wurden, beispielsweise auf 150 KeV (siehe Figuren 1 bis 5). Solche Elektronen sind niederenergetisch beschleunigt.

Das erfindungsgemäße Verfahren ist dadurch gekennzeichnet, dass die Elektronenstrahlen niederenergetisch oder mittelenergetisch mit einer Beschleunigungsenergie von zwischen 150 keV und 700 keV beschleunigt, mehr bevorzugt von zwischen 200 keV und 500 keV beschleunigt, noch mehr bevorzugt zwischen 250 keV und 400 keV beschleunigt werden.

Es ist möglich dabei unter Normaldruckatmosphäre, oder im Wesentlichen unter Normaldruckatmosphäre zu arbeiten und die Elektronenstrahlen können daher bevorzugt im Wesentlichen unter Normaldruckatmosphäre appliziert werden. Im Wesentlichen Normaldruckatmosphäre ist als 1 bar +/- 0,1 bar zu verstehen. Die Normaldruckatmosphäre kann dabei beispielsweise als Luftsauerstoff, Stickstoff oder Kohlendioxidgas vorliegen.

Wie in den Beispielen gezeigt konnte das PRRSV Virus unter Einsatz einer Dosis von 50 kGy, 100 kGy oder 200 kGy inaktiviert werden. Weiterhin konnte gezeigt werden, dass das Influenza A Virus in Suspension mit einer Dosis von 200 kGy komplett inaktiviert wird.

Die erfindungsgemäße Elektronenstrahldosis liegt im Bereich von 15 bis 110 kGy.

Es wurde gefunden, dass eine Dosis von mindestens 50 kGy vorteilhaft ist, um eine möglichst vollständige Inaktivierung der Viren zu erreichen.

Es wird offenbart, dass das Verfahren daher dadurch gekennzeichnet ist, dass die immunogene Zusammensetzung oder Vakzine enthaltend mindestens ein Virus mit einer Elektronenstrahldosis von mindestens 50 kGy, mindestens 60 kGy, mindestens 70 kGy, mindestens 80 kGy, mindestens 90 kGy, mindestens 100 kGy, mindestens 110 kGy, mindestens 120 kGy, mindestens 130 kGy, mindestens 140 kGy, mindestens 150 kGy, mindestens 160 kGy, mindestens 170 kGy, mindestens 180 kGy, mindestens 190 kGy, mindestens 200 kGy oder mindestens 250 kGy bestrahlt wird.

Weiterhin wurde gefunden, dass eine Dosis von 300 kGy oder weniger vorteilhaft ist, um mögliche Schädigungen der Zusammensetzung zu vermeiden.

Es wird offenbart, dass das Verfahren daher dadurch gekennzeichnet ist, dass die immunogene Zusammensetzung oder Vakzine enthaltend mindestens ein Virus mit einer Elektronenstrahldosis von höchstens 300 kGy, höchstens 250 kGy, höchstens 200 kGy, höchstens 190 kGy, höchstens 180 kGy, höchstens 170 kGy, höchstens 160 kGy, höchstens 150 kGy, höchstens 140 kGy, höchstens 130 kGy, höchstens 120 kgy, höchstens 110 kGy, höchstens 100 kGy, höchstens 90 kGy, höchstens 80 kGy, höchstens 70 kGy oder höchstens 60 kGy bestrahlt wird.

Es wird offenbart, dass die Elektronenstrahldosis im Bereich von 50 kGy bis 300 kGy liegt. Beispielsweise kann die immunogene Zusammensetzung oder Vakzine enthaltend mindestens ein Virus mit einer Elektronenstrahldosis von 50 kGy, 60 kGy, 70 kGy, 80 kGy, 90 kGy, 100 kGy, 110 kGy, 120 kGy, 130 kGy, 140 kgy, 150 kGy, 160 kGy, 170 kGy, 180 kGy, 190 kGy 200 kGy, 210 kGy, 220 kGy, 230 kGy, 230 kGy, 240 kGy, 250 kGy, 260 kGy, 270 kGy, 280 kGy, 290 kGy oder 300 kGy bestrahlt werden.

Es wird offenbart, dass das Verfahren daher dadurch gekennzeichnet ist, dass die immunogene Zusammensetzung oder Vakzine enthaltend mindestens ein Virus mit einer Elektronenstrahldosis im Bereich von 50 kGy bis 300 kGy, bevorzugt von 50 kGy bis 200 kGy, stärker bevorzugt 50 kGy bis 150 kGy, mehr bevorzugt von 50 kGy bis 120 kGy.

In einer bevorzugten Ausführungsform ist das erfindungsgemäße Verfahren daher dadurch gekennzeichnet ist, dass die immunogene Zusammensetzung oder Vakzine enthaltend mindestens ein Virus mit einer Elektronenstrahldosis im Bereich von 50 kGy bis 110 kGy bestrahlt wird.

Es wird offenbart, dass das mindestens eine Virus mit einer Elektronenstrahldosis von 1 bis 300 kGy, stärker bevorzugt mit einer Elektronenstrahldosis von 1 bis 150 kGy, noch stärker bevorzugt mit einer Elektronenstrahldosis von 10 bis 120 kGy bestrahlt wird.

Erfindungsgemäß wird das mindestens eine Virus mit einer Elektronenstrahldosis von 15 bis 110 kGy bestrahlt.

In Beispiel 1 konnte gezeigt werden, dass eine Inaktivierung um 2,5 log-Stufen durch Bestrahlen mit Elektronen mittels des erfindungsgemäßen Verfahrens erreicht werden konnte (Figur 1). Die Inaktivierung wurde durch Messen des TCID 50 Wertes bestimmt.

In einer bevorzugten Ausführungsform ist das erfindungsgemäße Verfahren daher dadurch gekennzeichnet, dass die Aktivität des mindestens einen Virus, bevorzugt gemessen als TCID50-Wert (50% infektiöse Dosis in Gewebekultur), nach Bestrahlung weniger als 5%, bevorzugt weniger als 1%, mehr bevorzugt weniger als 0,1% der Aktivität vor der Bestrahlung ist, noch mehr bevorzugt, dass nach Bestrahlung keine Aktivität des mindestens einen Virus mehr nachweisbar ist. Der TCID50-Wert kann daher wie in Beispiel 2 beschrieben bestimmt werden. Der Fachmann wird dabei eine geeignete Gewebekultur für einen bestimmten Virus auswählen. In Beispiel 2 wurde für das PRRSV Virus der TCID50-Wert in Marc145 Zellen bestimmt. In Beispiel 3 wurde für das Influenza A Virus der TCID50-Wert in MDCK-Zellen bestimmt.

Für das behüllte Virus PRRSV konnte in den Beispielen gezeigt werden, dass unter den angegebenen Bedingungen die Antigenstruktur im Wesentlichen erhalten blieb: die Bindung eines polyklonalen Serums gerichtet gegen das nicht-inaktivierte Virus an das mindestens eine Virus der bestrahlten Zusammensetzung entsprach über 90% der Bindung an das nicht-inaktivierte Virus (siehe Figur 1). Auch die Antigenstruktur von Influenza-Viren, die mit einer Elektronenstrahldosis von 200 kGy behandelt wurden, blieb im Wesentlichen erhalten (Figur 5). Die Bindung eines polyklonalen Serums aus einem Influenza-A-infizierten Menschen an das mit 200 kGy bestrahlte Virus entsprach ungefähr 80% der Bindung an das nicht-inaktivierte Virus.

In einer weiteren Ausführungsform ist das erfindungsgemäße Verfahren daher dadurch gekennzeichnet, dass das mindestens eine Virus ein behülltes Virus ist, und, dass die Antigenstruktur der Viren der immunogenen Zusammensetzung oder Vakzine nach Bestrahlung im Wesentlichen erhalten bleibt.

In einer anderen weiteren Ausführungsform ist das erfindungsgemäße Verfahren daher dadurch gekennzeichnet, dass das mindestens eine Virus ein unbehülltes Virus ist, und, dass die Antigenstruktur der Viren der immunogenen Zusammensetzung oder Vakzine nach Bestrahlung im Wesentlichen erhalten bleibt.

Antigene sind Stoffe, wie insbesondere Proteine, an die Antikörper spezifisch binden können. Um als Antigene zu wirken müssen die Antigene, bzw. die darin enthaltenen Epitope chemisch und strukturell intakt sein. Dabei ist häufig der Erhalt der zwei- und/oder drei-dimensionalen Struktur der Antigene, bzw. der darin enthaltenen Epitope für die Bindung der Antikörper erforderlich. Überraschenderweise wurde nun gefunden, dass durch Bestrahlung mit Elektronenstrahlen die Antigenstruktur der bestrahlten Viren im Wesentlichen erhalten bleibt und somit die bestrahlte flüssige Zusammensetzung als Vakzine eingesetzt werden kann, um eine spezifische Immunantwort in einem Menschen oder Tier, insbesondere Säugetier, auszulösen.

Ein Epitop ist ein Bereich eines Antigens, an den ein Antikörper spezifisch bindet.

In einer besonders bevorzugten Ausführungsform ist dabei die Bindung eines polyklonalen Serums gerichtet gegen das nicht-inaktivierte Virus an das mindestens eine Virus der bestrahlten immunogenen Zusammensetzung oder Vakzine mindestens 40%, bevorzugt mindestens 70%, mehr bevorzugt mindestens 80%, noch mehr bevorzugt mindestens 90% der Bindung des polyklonalen Serums an das mindestens eine Virus der immunogenen Zusammensetzung oder Vakzine vor der Bestrahlung.

Die Bindung des polyklonalen Serums an das mindestens eine Virus der immunogenen Zusammensetzung oder Vakzine wird dabei bevorzugt mittels ELISA bestimmt. Die Bestimmung der Bindung mittels ELISA wird dabei bevorzugt wie in den Beispielen dargelegt durchgeführt.

ELISA (Enzyme Linked Immunosorbent Assay) bezeichnet ein antikörperbasiertes Nachweisverfahren, das dem Fachmann seit Jahrzehnten sehr gut bekannt ist. Mit Hilfe des ELISA können Substanzen wie beispielsweise Proteine nachgewiesen werden. Hierbei macht man sich die Eigenschaft spezifischer Antikörper zunutze, die an die nachzuweisende Substanz, das Antigen, binden. Ein Antikörper wird zuvor mit einem Enzym markiert. Die durch das Reporterenzym katalysierte Reaktion dient als Nachweis für das Vorhandensein des Antigens. Ein Substrat wird vom Enzym umgesetzt und das Reaktionsprodukt kann dann nachgewiesen werden, beispielsweise durch Messung der Absorption oder Chemolumineszenz.

In einer weiteren Ausführungsform ist das erfindungsgemäße Verfahren daher dadurch gekennzeichnet, dass das mindestens eine Virus ein behülltes Virus ist, und dass die Antigenstruktur der Viren der Zusammensetzung nach Bestrahlung im Wesentlichen erhalten bleibt.

In einer anderen weiteren Ausführungsform ist das erfindungsgemäße Verfahren daher dadurch gekennzeichnet, dass das mindestens eine Virus ein unbehülltes Virus ist, und dass die Antigenstruktur der Viren der Zusammensetzung nach Bestrahlung im Wesentlichen erhalten bleibt.

In einer anderen Ausführungsform ist die Bindung eines polyklonalen Serums gerichtet gegen das nicht-inaktivierte Virus an das mindestens eine Virus der bestrahlten Zusammensetzung mindestens 40%, bevorzugt mindestens 70%, mehr bevorzugt mindestens 80%, noch mehr bevorzugt mindestens 90% der Bindung des polyklonalen Serums an das mindestens eine Virus der Zusammensetzung vor der Bestrahlung.

Die Bindung des polyklonalen Serums an das mindestens eine Virus der Zusammensetzung wird dabei bevorzugt mittels ELISA bestimmt. Die Bestimmung der Bindung mittels ELISA wird dabei bevorzugt wie in den Beispielen dargelegt durchgeführt.

Es ist weiterhin vorteilhaft für die Herstellung von immunogenen Zusammensetzungen und Vakzinen (Impfstoffe) für unbehüllte und behüllte Viren, wenn die Virusstruktur der Viren nach Bestrahlung im Wesentlichen erhalten bleibt. Dies gilt insbesondere wenn ein Ganzkörperimpfstoff, insbesondere inaktivierter Ganzkörperimpfstoff, gewünscht ist. Ganzkörperimpfstoffe haben den Vorteil, dass diese die verschiedenen Antigene des Virus enthalten und somit eine umfassende Immunantwort auslösen können. In den Beispielen konnte für ein behülltes Virus gezeigt werden, dass die Bindung eines Antikörpers gerichtet gegen ein Antigen des Virus, das bei intakter Hülle dieses Virus für den Antikörper nicht zugänglich ist, nämlich das N-Protein des PRRSV, bei der bestrahlten Zusammensetzung und der der negativen Kontrolle im wesentlichen gleich ist, während in der Peroxidbehandelten Zusammensetzung ein Anstieg der Bindung von über 400% festgestellt wurde.

Ein Ganzkörperimpfstoff oder Ganzpartikelimpfstoff ist ein Impfstoff enthaltend ein Virus, dessen Partikelstruktur im Wesentlichen erhalten ist.

Ein inaktivierter Ganzkörperimpfstoff oder Ganzpartikelimpfstoff ist ein Totimpfstoff enthaltend ein Virus, dessen Virusstruktur im Wesentlichen erhalten ist.

Ein Totimpfstoff enthält inaktivierte oder abgetötete Viren oder Bakterien oder Bestandteile von Viren, oder Bakterien oder Giftstoffen. Diese können sich im Körper nicht mehr weitervermehren.

Ein Immunogen gemäß der vorliegenden Erfindung ist ein Antigen, das aufgrund seiner Immunogenität in der Lage ist, eine Immunantwort auszulösen.

Eine immunogene Zusammensetzung ist eine Zusammensetzung, die in der Lage ist, eine Immunantwort in einem Menschen oder Säugetier auszulösen. Eine immunogene Zusammensetzung enthält mindestens ein Immunogen. In einer bevorzugten Ausführungsform ist eine immunogene Zusammensetzung geeignet zur Verabreichung an den Menschen oder ein Tier und ist somit zur Gabe an ein Tier oder Menschen hergerichtet. Die Zusammensetzung enthält daher bevorzugt keine Stoffe, die nicht zur Verabreichung an den Menschen oder ein Säugetier zugelassen oder dafür geeignet sind, insbesondere keine krebserregenden, stark allergieauslösenden oder toxischen Substanzen.

Eine Vakzine oder Impfstoff gemäß der vorliegenden Erfindung enthält mindestens ein Antigen und/oder Immunogen, das aufgrund seiner Immunogenität in der Lage ist, eine Immunantwort auszulösen, und ist zur Gabe an ein Tier oder Menschen hergerichtet. Eine Vakzine ist geeignet zur Verabreichung an den Menschen oder ein Tier. Eine Vakzine enthält daher keine Stoffe, die nicht zur Verabreichung an den Menschen oder ein Säugetier zugelassen oder dafür geeignet sind, insbesondere keine krebserregenden, stark allergieauslösenden oder toxischen Substanzen.

Ein Impfstoff oder eine Vakzine gerichtet gegen ein Virus schützt bevorzugt gegen eine virale Infektion oder Erkrankung mildert diese ab. Ein Impfstoff oder eine Vakzine gerichtet gegen ein Virus ist daher bevorzugt zur Prävention und/oder Behandlung, wie beispielsweise Abmilderung, einer viralen Infektion oder Erkrankung in einem Menschen oder Tier, insbesondere Säugetier, geeignet.

In einer bevorzugten Ausführungsform ist das erfindungsgemäße Verfahren daher dadurch gekennzeichnet, dass die Virusstruktur der Viren nach Bestrahlung im Wesentlichen erhalten bleibt.

In einer bevorzugten Ausführungsform ist das erfindungsgemäße Verfahren daher dadurch gekennzeichnet, dass das mindestens eine Virus ein behülltes Virus ist, und dass die Virusstruktur der Viren nach Bestrahlung im Wesentlichen erhalten bleibt.

In einer weiteren bevorzugten Ausführungsform ist das erfindungsgemäße Verfahren daher dadurch gekennzeichnet, dass das mindestens eine Virus ein unbehülltes Virus ist, und dass die Virusstruktur der Viren nach Bestrahlung im Wesentlichen erhalten bleibt.

Eine Virusstruktur bleibt im Wesentlichen erhalten im Sinne der vorliegenden Erfindung, wenn die Bindung eines Antikörpers gerichtet gegen ein Antigen des Virus, das bei intakter Hülle dieses Virus für den Antikörper nicht zugänglich ist, an den mindestens einen Virus der Zusammensetzung, insbesondere immunogenen Zusammensetzung oder Vakzine, nach Bestrahlung weniger als 400%, bevorzugt weniger als 200%, mehr bevorzugt weniger als 150%, noch mehr bevorzugt weniger als 120% der Bindung dieses Antikörpers an den mindestens einen Virus der Zusammensetzung, insbesondere immunogenen Zusammensetzung oder Vakzine, vor Bestrahlung ist. Dies wird bevorzugt durch ELISA bestimmt, wie in den Beispielen gezeigt. In Figur 3 wurde überraschenderweise gezeigt, dass bei Bestrahlung einer flüssigen immunogenen Zusammensetzung enthaltend PRRSV Viren das Capsid (N-) Protein in einem ELISA unter Verwendung eines Antikörpers gegen dieses Antigen nicht zugänglich wird.

In einer weiteren Ausführungsform betrifft daher die vorliegende Erfindung außerdem die Verwendung eines erfindungsgemäßen Verfahrens zur Herstellung eines inaktivierten viralen Ganzpartikelimpfstoffs.

Bei den erfindungsgemäßen Verfahren kommen Elektronenstrahlen zum Einsatz. Bei der technischen Erzeugung von Elektronen bestimmt - nach Stromeinschaltung - die bei deren Emission angelegte Beschleunigungsspannung zwischen Kathode und Anode ihren Energiegehalt in Elektronenvolt eV. Je höher dieser ist, desto tiefer kann das mit mehr Energie versehene Elektron in Materie hineinstoßen. Die Strahlungsleistung ist das Produkt aus Strahlstrom (Menge der erzeugten Elektronen) und Beschleunigungsspannung - angegeben in Kilowatt (kW). Bei hohen Strahlungsleistungen kann die erforderliche Energiedosis zur Auslösung strahlenchemischer Reaktionen in sehr kurzen Zeiten (Bruchteilen von Sekunden) erzeugt und appliziert werden. Insgesamt sind Elektronenstrahlen, trotz geringerer Eindringtiefe, insbesondere bei Durchlaufprozessen hoher Produktmengen im Vorteil: es lassen sich technisch gut justierbar physikalisch-chemische Prozesse initiieren und umsetzen. Ein wesentlicher Vorteil ist, dass der Strahlungsprozess praktisch per Knopfdruck ein- oder ausgeschaltet werden kann (beispielsweise im Gegensatz zu Gamma-Strahlen).

Geeignete Vorrichtungen zur Erzeugung von Elektronenstrahlen und Vorrichtungen zur Durchführung des Verfahrens sind im Stand der Technik bekannt (A. Heger: Technologie der Strahlenchemie von Polymeren, Carl Hanser Verlag München Wien, 1990. ISBN 3-446-15630-5; Kapitel 2: Allgemeine Grundlagen; S. 25-39 und Kapitel 4: Industrielle Bestrahlungsanlagen; S. 69-149; DE 196 389 25 A1).

Elektronenstrahlen sind auch gegenüber Gamma-Strahlen vorteilhaft: Da übliche Gamma-Quellen oft nur ca. 2 bis 6 kGy pro Stunde liefern, sind höhere Dosen nur in langen Zeitverläufen realisierbar, während Elektronenstrahler dies in wenigen Sekunden erreichen (hohe Dosisraten) und somit wesentlich produktiver sind.

Bei üblicher Gamma-Bestrahlung an Luft in kommerziellen Dienstleistungsanlagen kommt eine langzeitbegleitende oxidative Wirkung (Ozon bei Luft oder OH-Radikale von Wasser) auf das behandelte Material bzw. zum Reaktionsprozess hinzu, was oft nicht bzw. gelegentlich doch Produktveränderungen bzw. -ergebnisse im erwünschten Sinne bringt. Bei kurzem Dosisstoß mittels Elektronenstrahlanlagen, die zumeist auch auf Arbeiten unter Inertatmosphäre ausgelegt sind, werden solche Nebenwirkungen über längere Einflusszeit ausgeschlossen.

Bevorzugt wird das erfindungsgemäße Verfahren unter Verwendung einer Vorrichtung zur Erzeugung von Elektronenstrahlen durchgeführt, die kontinuierlich oder schnell gepulst arbeitet.

In einer weiteren bevorzugten Ausführungsform wird das erfindungsgemäße Verfahren unter Verwendung einer Vorrichtung zur Erzeugung von Elektronenstrahlen durchgeführt, die nach dem Kalt- oder Glühkathodenprinzip Elektronen liefert.

In einer weiteren bevorzugten Ausführungsform wird das erfindungsgemäße Verfahren unter Verwendung einer Vorrichtung zur Erzeugung von Elektronenstrahlen durchgeführt, die als Axialstrahler (Scanner) oder Linear-Breitbandstrahler ausgeführt ist.

In einer weiteren bevorzugten Ausführungsform des erfindungsgemäßen Verfahrens werden die Elektronen nach Austritt aus der evakuierten Erzeugerkammer der Vorrichtung durch ein Austrittsfenster auf die Zusammensetzung appliziert. Dabei ist die immunogene Zusammensetzung oder Vakzine bevorzugt in einem Container.

In einer weiteren bevorzugten Ausführungsform des erfindungsgemäßen Verfahrens wird die immunogene Zusammensetzung oder Vakzine statisch in die Vorrichtung aufgenommen, oder kontinuierlich durch den Elektronenstrahl transportiert.

Weiterhin kann die Dosisrate (Strahlstrom/Zeit) angepasst werden. Dabei ist generell zu berücksichtigen, dass - in Bezug auf die gewünschte finale, applizierte Dosis - ein hoher Strahlstrom wenig Bestrahlungszeit erfordert, und wenig Strahlstrom eine lange Bestrahlungszeit erfordert. Die Dosisrate wird vom Fachmann angepasst unter Berücksichtigung z.B. beim Spaltrohr der Durchströmgeschwindigkeit des Flüssigmediums, sowie des Strahlertyps.

Beispielsweise kann eine Dosis im Bereich von 10 bis 300 kGy, insbesondere von 50 bis 300 kGy, in einer Zeit von bis zu 1000 sek appliziert werden; niedrige Dosen von 1 bis 25 kGy können in einer Zeit von 0,1 bis 100 Sekunden appliziert werden. Dosen ab 50 kGy, die bevorzugt sind, benötigen typischerweise eine Applikationszeit von 10 Sekunden bis 1000 Sekunden. Daher beträgt die Applikationszeit in einer Offenbarung 10 Sekunden bis 1000 Sekunden.

In einer weiteren bevorzugten Ausführungsform des erfindungsgemäßen Verfahrens ist daher die Dosisrate im Bereich von 1 kGy/0,1 Sekunden bis 150 kGy/1000 sek.

In dem erfindungsgemäßen Verfahrens ist daher die Bestrahlungszeit im Bereich von zwischen 1 Sekunde und 100 Sekunden.

Offenbart wird, dass die Bestrahlungszeit im Bereich von 0,1 Sekunden bis 1000 Sekunden liegt.

Bei der Bestrahlung findet typischerweise eine Temperaturerhöhung statt. Zur Vermeidung von Denaturierungsprozessen ist es daher von Vorteil, wenn die Temperatur nur gering ansteigt.

In einer weiteren bevorzugten Ausführungsform des erfindungsgemäßen Verfahrens ist daher die Temperatur immunogenen Zusammensetzung oder Vakzine vor der Bestrahlung zwischen 1°C und 40°C, vorzugsweise zwischen 5°C und 37°C, stärker bevorzugt zwischen 10°C und 32°C, noch stärker bevorzugt zwischen 15°C und 30°C.

Es ist in einer anderen Ausführungsform möglich die Bestrahlung bei Temperaturen der Zusammensetzung vor der Bestrahlung von weniger als 1°C durchzuführen, beispielsweise von gefrorenen Zusammensetzungen. In diesem Fall kann die Zusammensetzung nach Bestrahlung ebenfalls eine Temperatur von weniger als 1°C aufweisen, oder die Temperatur der Zusammensetzung nach Bestrahlung kann 1°C oder mehr betragen.

In einer weiteren bevorzugten Ausführungsform des erfindungsgemäßen Verfahrens ist die Temperaturerhöhung der immunogenen Zusammensetzung oder Vakzine nach der Bestrahlung im Vergleich zu vor der Bestrahlung zwischen 1°C und 15°C, vorzugsweise zwischen 2°C und 10°C.

In einer weiteren bevorzugten Ausführungsform des erfindungsgemäßen Verfahrens ist daher die Temperatur der immunogenen Zusammensetzung oder Vakzine nach der Bestrahlung zwischen 2°C und 41°C, vorzugsweise zwischen 6°C und 38°C, stärker bevorzugt zwischen 11°C und 33°C, noch stärker bevorzugt zwischen 16°C und 31°C.

Die immunogene Zusammensetzung oder Vakzine enthaltend mindestens ein Virus ist flüssig. Dabei kann eine solche Flüssigkeit bevorzugt als Suspension von Viren in einer wässrigen Lösung vorliegen, wie in den Beispielen; es kann jedoch auch eine Suspension höherer Dichte vorliegen.

In einer weiteren bevorzugten Ausführungsform des erfindungsgemäßen Verfahrens ist daher die Dichte der immunogenen Zusammensetzung oder Vakzine zwischen 0,9 und 2 g/cm³, bevorzugt zwischen 1,0 und 1,8 g/cm³.

In einer weiteren bevorzugten Ausführungsform des erfindungsgemäßen Verfahrens ist daher die immunogene Zusammensetzung oder Vakzine enthaltend mindestens ein Virus eine flüssige Suspension enthaltend Wasser, bevorzugt eine Suspension des mindestens ein Virus in einer wässrigen Lösung, wobei die wässrige Lösung besonders bevorzugt eine oder mehrere Puffersubstanzen enthält. Die wässrige gepufferte Lösung kann beispielsweise PBS sein. Der pH einer solchen Lösung ist bevorzugt im Bereich von pH 5,5 bis 8,5, stärker bevorzugt im Bereich von pH 6,5 bis 8,0.

Es ist möglich mit dem erfindungsgemäßen Verfahren eine ansonsten fertige Vakzine zu bestrahlen, die bereits geeignete Hilfsstoffe und/oder Adjuvantien erhält.

In einer weiteren Ausführungsform des erfindungsgemäßen Verfahrens enthält daher die immunogene Zusammensetzung oder Vakzine (a) einen oder mehrere Adjuvantien, und/oder (b) pharmazeutisch annehmbare Träger- und/oder Hilfsstoffe und/oder (c) ein oder mehrere weitere Immunogene.

In einer weiteren bevorzugten Ausführungsform des erfindungsgemäßen Verfahrens besteht daher die immunogene Zusammensetzung oder Vakzine aus mindestens einem Virus, insbesondere einem (1) Virus, und
(a) einem oder mehrere Adjuvantien, und/oder
(b) pharmazeutisch annehmbare Träger- und/oder Hilfsstoffen, wie beispielsweise Wasser oder eine geeignete wässrige Lösung, die besonders bevorzugt eine oder mehrere Puffersubstanzen enthält,
und (c) optional einem oder mehreren weitere Viren oder Immunogenen.

In einer weiteren Ausführungsform des erfindungsgemäßen Verfahrens ist daher die Zusammensetzung eine Vakzine enthaltend mindestens ein virales Immunogen und optional enthaltend (a) einen oder mehrere Adjuvantien, und/oder (b) pharmazeutisch annehmbare Träger- und/oder Hilfsstoffe und/oder (c) ein oder mehrere weitere Immunogene.

Geeignete Träger- und Hilfsstoffe und/oder weitere Adjuvantien sind dem Fachmann gut bekannt. Geeignete Adjuvantien sind solche, die ausreichend sind um eine Immunantwort auf ein Immunogen zu verstärken. Geeignete Adjuvantien sind beispielsweise Aluminiumsalze wie Aluminiumphosphat oder Aluminiumhydroxid, Squalenmischungen (SAF-1), Muramyl-Peptid, Saponin-Derivate, Mycobacterium Zellwand-Zubereitungen, Monophosphoryl-Lipid A, Mykolsäure Derivate, nichtionische Block-Copolymer grenzflächenaktive Substanzen, Quil A, Cholera-Toxin B Untereinheit, Polyphosphazen und Derivate und immunstimulierende Komplexe (ISCOMs) wie solche die in Takahashi et al. (1990) Nature 344:873-875 beschrieben sind.

Geeignete Träger- und Hilfsstoffe sind beispielsweise Wasser oder eine zur Verabreichung geeignete wässrige Lösung, die besonders bevorzugt eine oder mehrere Puffersubstanzen enthält.

Geeignete weitere Immunogene sind dem Fachmann gut bekannt und umfassen insbesondere
(a) organische Substanzen, insbesondere Proteine, die glykosyliert oder unglykosyliert sein können, Nukleinsäuren, Toxine, oder Zuckermoleküle, insbesondere Saccharide, die optional an einen Träger gebunden sein können, und
(b) ein Virus oder ein Lebewesen, insbesondere ein Bakterium, wobei das Virus oder Lebewesen aktiv oder inaktiviert sein kann.

Geeignete weitere Immunogene sind insbesondere solche, die eine Immunantwort auf einen Krankheitserreger oder Krankheitsfaktor im gleichen Tier oder im Menschen auslösen, wie das mindestens eine Virus der Zusammensetzung. Ist beispielsweise das mindestens eine Virus ein humanpathogenes Virus, so sind weitere Immunogene bevorzugt so auszuwählen, dass sie eine Immunantwort auf einen humanpathogenen Erreger auslösen und/oder eine humane Krankheit vorbeugen oder abmildern.

Bei lyophilisierten Vakzinen können stabilisierende Agenzien als Träger- und Hilfsstoffe zugegeben werden, beispielsweise ein Polyol wie Saccharose oder Trehalose.

Wie bei allen immunogenen Zusammensetzungen oder Vakzinen üblich, muss die immunologisch wirksame Dosis empirisch bestimmt werden. Faktoren, die dabei berücksichtigt werden sollten sind, ob ein Immunogen mit einem Adjuvans oder Trägermolekül komplexiert werden soll oder kovalent an dieses gebunden werden soll, die Art der Gabe, und die Anzahl der immunisierenden Dosen die gegeben werden sollen. Solche Faktoren sind im Gebiet der Vakzinentwicklung gut bekannt und der Fachmann in diesem Gebiet kann diese Faktoren ohne weiteres bestimmen.

Das mindestens eine Virus und gegebenenfalls ein oder mehrere weitere Immunogene können in verschiedenen Konzentrationen in immunogenen Zusammensetzungen oder Vakzinen enthalten sein. Die minimale Konzentration in einer Vakzine ist üblicherweise diejenige, die für ihre geplante Verwendung zur Impfung ausreichend ist, wobei bei der Bestrahlung gemäß dem erfindungsgemäßen Verfahren auch geringere Konzentrationen eingesetzt werden können, und eine höhere Konzentration dann in der fertigen Vakzine eingestellt werden kann oder bei der Bestrahlung gemäß dem erfindungsgemäßen Verfahren auch höhere Konzentrationen eingesetzt werden können, und eine geringere Konzentration dann in der fertigen Vakzine eingestellt werden kann. Die maximale Konzentration für die Bestrahlung gemäß dem erfindungsgemäßen Verfahren ist üblicherweise eine solche, bei der das mindestens eine Virus während der Bestrahlung homogen suspendiert bleibt und/oder gegebenenfalls ein oder mehrere weitere Immunogene gelöst oder während der Bestrahlung homogen suspendiert bleiben. Die maximale Konzentration in einer Vakzine ist üblicherweise eine solche, bei der das mindestens eine inaktivierte Virus homogen suspendiert bleibt und/oder gegebenenfalls ein oder mehrere weitere Immunogene gelöst oder homogen suspendiert bleiben.

Die Vakzine können verwendet werden, um einen Menschen oder ein Tier, insbesondere ein Säugetier, durch Verabreichung, insbesondere durch systemische Gabe oder Gabe über die Schleimhaut zu schützen oder zu behandeln. Die Art der Gabe kann vom Fachmann gewählt werden und umfasst beispielsweise die Injektion über den intramuskulären, intraperitonealen, intradermalen oder subkutanen Weg, oder die Gabe über die Schleimhaut zum oralen, respiratorischen oder geniurinären Trakt.

Die Herstellung von Vakzinen ist allgemein beschrieben in Vaccine Design ("The subunit and adjuvant approach" (Hsg. Powell M. F. & Newman MJ.) (1995) Plenum Press New York).

Es ist alternativ aber auch möglich, zunächst eine immunogene Zusammensetzung enthaltend mindestens ein Virus erfindungsgemäß zu bestrahlen, und anschließend gegebenenfalls geeignete Hilfsstoffe und/oder Adjuvantien zuzugeben. Es können auch weitere Immunogene zur Herstellung von Kombinationsimpfstoffen zugegeben werden.

In einer weiteren Ausführungsform betrifft die Erfindung daher ein Verfahren zur Herstellung einer Vakzine, enthaltend mindestens ein virales Immunogen, insbesondere einer Vakzine, umfassend einen viralen Ganzpartikelimpfstoff, dadurch gekennzeichnet, dass:
(a) das erfindungsgemäße Verfahren wie oben beschrieben durchgeführt wird,
(b1) der Zusammensetzung, insbesondere immunogenen Zusammensetzung, enthaltend mindestens einen Virus optional ein oder mehrere Adjuvantien zugegeben werden, und/oder
(b2) der Zusammensetzung, insbesondere immunogenen Zusammensetzung, enthaltend mindestens einen Virus optional ein oder mehrere pharmazeutisch annehmbare Träger- und/oder Hilfsstoffe zugegeben werden, und/oder
(b3) der Zusammensetzung, insbesondere immunogenen Zusammensetzung, enthaltend mindestens einen Virus optional ein oder mehrere weitere Immunogene zugegeben werden,
wobei die Schritte (a) bis (b3) in beliebiger Reihenfolge durchgeführt werden.

Um für die Applikation an ein Tier oder einen Menschen geeignet zu sein, und um einen sicheren Transport und eine Applikation in definierter Dosis zu gewährleisten, ist eine solche Vakzine üblicherweise steril und in einem geeigneten Container abgefüllt. Ein solcher Container kann mehrere Dosen oder Einzeldosen enthalten.

In einer weiteren bevorzugten Ausführungsform ist das erfindungsgemäße Verfahren zur Herstellung einer Vakzine dadurch gekennzeichnet, dass folgende weitere Schritte durchgeführt werden:
(c) Sterilisieren der immunogenen Zusammensetzung, und/oder
(d) Abfüllen der immunogenen Zusammensetzung in einen Container,
wobei die Schritte (a) bis (d) in beliebiger Reihenfolge durchgeführt werden können, und anschließend an die Schritte (a) bis (d) die Vakzine optional getrocknet, gefriergetrocknet oder eingefroren wird.

In einer weiteren Ausführungsform des erfindungsgemäßen Verfahrens wird daher die Zusammensetzung enthaltend mindestens ein Virus darüber hinaus
(c) sterilisiert, und/oder
(d) in Container abgefüllt,
wobei die Schritte (a) bis (d) in beliebiger Reihenfolge durchgeführt werden können, und anschließend die Vakzine optional getrocknet, gefriergetrocknet oder eingefroren wird.

Die mit dem erfindungsgemäßen Verfahren erhaltenen immunogenen Zusammensetzungen und Vakzine sind den Zusammensetzungen im Stand der Technik klar überlegen, da sie keine Reste an chemischen Inaktivierungssubstanzen (wie Formaldehyd) enthalten und/oder durch intakte Antigenstruktur des Virus bei gleichzeitiger Inaktivierung des mindestens einen Virus gekennzeichnet sind. Dies ist insbesondere bei einem Ganzpartikelimpfstoff und/oder Totimpfstoff der Fall.

Daher wird weiterhin eine immunogene Zusammensetzung oder Vakzine, bevorzugt Vakzine, besonders bevorzugt enthaltend einen inaktivierten viralen Ganzpartikelimpfstoff, herstellbar durch ein erfindungsgemäßes Verfahren offenbart.

Hierbei handelt es sich um eine immunogene Zusammensetzung oder Vakzine, bevorzugt Vakzine, enthaltend einen inaktivierten viralen Ganzpartikelimpfstoff für einen behüllten oder unbehüllten Virus, bevorzugt behüllten Virus, dadurch gekennzeichnet, dass
(a) die Aktivität des Virus in der immunogenen Zusammensetzung oder Vakzine weniger als 10%, bevorzugt weniger als 1%, mehr bevorzugt weniger als 0,1% der Aktivität der gleichen Anzahl nicht-inaktivierter Viren ist, und
(b) die Antigenstruktur der inaktivierten Viren in der immunogenen Zusammensetzung oder Vakzine im Wesentlichen gleich im Vergleich zur gleichen Anzahl nicht-inaktivierter Viren ist.

In einer bevorzugten Ausführungsform dieser immunogenen Zusammensetzung oder Vakzine ist die Virusstruktur der inaktivierten Viren im Wesentlichen gleich im Vergleich zur gleichen Anzahl nicht-inaktivierter Viren.

Diese immunogene Zusammensetzung oder Vakzine wurde mit einem Elektronenstrahl bestrahlt, wie oben beschrieben.

Eine Elektronenstrahldosis von 50 kGy bis 300 kGy, bevorzugt von 50 kGy bis 200 kGy, stärker bevorzugt 50 kGy bis 150 kGy, mehr bevorzugt von 50 kGy bis 120 kGy, noch mehr bevorzugt von 50 kGy bis 110 kGy erlaubt eine effektive Inaktivierung eines Virus, wobei gleichzeitig die Virusstruktur als auch Antikörperstruktur eines behüllten oder unbehüllten Virus, bevorzugt behüllten Virus, erhalten bleibt. Die erfindungsgemäße Strahlendosis liegt zwischen 15 bis 110kGy.

In einer weiteren bevorzugten Ausführungsform ist keine Aktivität des mindestens einen Virus in der Zusammensetzung, insbesondere immunogenen Zusammensetzung oder Vakzine, mehr nachweisbar. Dies ist insbesondere für einen Totimpfstoff und/oder inaktivierten viralen Ganzkörperimpfstoff von Bedeutung.

Die durch das erfindungsgemäße Verfahren hergestellte immunogene Zusammensetzung, bevorzugt Vakzine, ist geeignet zur Verwendung als Vakzine, insbesondere zur Prävention oder Behandlung, insbesondere Abmilderung, von viralen Infektionen oder Erkrankungen, die durch das Virus verursacht werden.

Die Erfindung betrifft in einer weiteren Ausführungsform die Verwendung von Elektronenstrahlen zur Inaktivierung von Viren in einer immunogenen Zusammensetzung oder Vakzine enthaltend mindestens ein Virus, wobei die immunogene Zusammensetzung oder Vakzine (i) flüssig ist, insbesondere eine Suspension ist, und (ii) mindestens ein virales Immunogen enthält, wobei die Elektronenstrahldosis im Bereich von 15 bis 110 kGy und die Bestrahlungszeit im Bereich von zwischen 1 Sekunde und 100 Sekunden ist und die Elektronenstrahlen mit einer Beschleunigungsenergie von zwischen 150 keV und 700 keV beschleunigt werden.

Offenbart wird die Verwendung von Elektronenstrahlen zur Inaktivierung von Viren in flüssigen Zusammensetzungen, bevorzugt flüssigen immunogenen Zusammensetzungen oder Vakzinen, stärker bevorzugt Vakzinen.

Die Erfindung betrifft in einer weiteren Ausführungsform die Verwendung von Elektronenstrahlen zur Herstellung eines inaktivierten viralen Ganzpartikelimpfstoffs wobei die Elektronenstrahldosis im Bereich von 15 bis 110 kGy und die Bestrahlungszeit im Bereich von zwischen 1 Sekunde und 100 Sekunden ist und die Elektronenstrahlen mit einer Beschleunigungsenergie von zwischen 150 keV und 700 keV beschleunigt werden.

Die Elektronenstrahlen werden niederenergetisch oder mittelenergetisch mit einer Beschleunigungsenergie von zwischen 150 keV und 700 keV beschleunigt, mehr bevorzugt von zwischen 200 keV und 500 keV beschleunigt, noch mehr bevorzugt zwischen 250 keV und 400 keV beschleunigt und/oder im Wesentlichen unter Normaldruckatmosphäre appliziert, bevorzugt wobei die Normaldruckatmosphäre als Luftsauerstoff, Stickstoff oder Kohlendioxidgas vorliegt.

In einer bevorzugten Ausführungsform der erfindungsgemäßen Verwendung liegt die Elektronenstrahldosis im Bereich von 50 kGy bis 110 kGy.

Offenbart wird die Verwendung einer Vorrichtung zur Erzeugung von Elektronenstrahlen zur Inaktivierung von Viren in flüssigen Zusammensetzungen, bevorzugt flüssigen immunogenen Zusammensetzungen, stärker bevorzugt Vakzinen, insbesondere flüssigen Vakzinen.

Offenbart wird die Verwendung einer Vorrichtung zur Erzeugung von Elektronenstrahlen zur Herstellung eines inaktivierten viralen Ganzpartikelimpfstoffs.

In einer bevorzugten Ausführungsform der erfindungsgemäßen Verwendungen ist die Vorrichtung zur Emission niederenergetisch oder mittelenergetisch beschleunigter Elektronenstrahlen geeignet, bevorzugt zur Emission beschleunigter Elektronenstrahlen mit einer Beschleunigungsenergie von zwischen 150 keV und 700 keV geeignet, mehr bevorzugt zur Emission beschleunigter Elektronenstrahlen mit einer Beschleunigungsenergie von zwischen 200 keV und 500 keV geeignet, noch mehr bevorzugt zur Emission beschleunigter Elektronenstrahlen mit einer Beschleunigungsenergie von zwischen 250 keV und 400 keV geeignet.

In einer weiteren bevorzugten Ausführungsform der erfindungsgemäßen Verwendungen ist die Vorrichtung zur Applikation von Elektronenstrahlen im Wesentlichen unter Normaldruckatmosphäre geeignet.

In einer weiteren bevorzugten Ausführungsform der erfindungsgemäßen Verwendungen ist die Vorrichtung zur Abgabe einer Elektronenstrahldosis von 50 kGy bis 110 kGy geeignet.

In einer weiteren Ausführungsform der erfindungsgemäßen Verwendungen ist die Vorrichtung zur Abgabe einer Elektronenstrahldosis von 15 bis 110 kGy geeignet.

Die bevorzugten Ausführungsformen für das erfindungsgemäße Verfahren gelten auch für die erfindungsgemäßen Verwendungen und immunogenen Zusammensetzungen und Vakzinen, soweit anwendbar.

### Figuren:

- Figur 1:: zeigt die PRRS-Virus-Aktivität nach Behandlung mit dem Elektronenstrahl (grau) und ohne Bestrahlung (schwarz). Angegeben sind Werte der TCID50 pro mL Viruslösung. Dosis: 100 kGy.
- Figur 2:: zeigt die Ergebnisse zur Antigen-Unversehrtheit nach Behandlung. PRRS-Viren wurden mit Elektronenstrahl (grau), Formaldehyd (Streifen) oder gar nicht (schwarz) behandelt. Die Unversehrtheit der Antigene wurde über Inkubation mit einem polyklonalen Serum aus einem PRRSV-Infizierten Schwein bestimmt. Angegeben sind OD-Werte (Optische Dichte) im ELISA. Dosis: 100 kGy.
- Figur 3:: zeigt die Ergebnisse Unversehrtheit der Virushülle nach Behandlung. PRRS-Viren wurden mit Elektronenstrahl (grau), Peroxid (Streifen) oder gar nicht (schwarz) behandelt. Die Unversehrtheit der Virushülle wurde mit einem Antikörper gegen das Capsid- (N-) Protein von PRRSV analysiert. Angegeben sind OD-Werte im ELISA. Dosis: 100 kGy.
- Figur 4:: H3N8-Viren wurden mit niederenergetischen Elektronen von 200 kGy (E) oder 0 kGy (NC) behandelt und ihre Aktivität über eine TCID50-Bestimmung gemessen.
- Figur 5:: Die Unversehrtheit der Antigene wurde über Inkubation mit einem Serum aus einem Influenza-positiven Menschen bestimmt. Werte sind Absorptions-Signale in einem ELISA-Test. E: H3N8-Viren mit einer Elektronenstrahldosis von 200 kGy behandelt; NC: H3N8-Viren mit 0 kGy behandelt (Kontrolle).

### Beispiel 1: Zusammenfassung der Experimente

Die Versuche wurden beispielhaft mit dem PRRS-Virus (porcine respiratory and reproductive failure syndrome virus) durchgeführt. Dieses Virus ist ein einzelsträngiges, positiv-Strang-RNA Virus aus der Familie *Arteriviridae.* Das Virus befällt Schweine und verursacht jährliche Schäden in der Schweineindustrie in Milliarden-Höhe. PRRSV wurde in 100 µL flüssigem Medium durch den Elektronenstrahl geführt und mit 100 kGy bestrahlt. Die eingesetzte Virus-Menge waren 2*10⁴ TCID50 pro Ansatz. Danach wurde die Aktivität der Erreger sowie die Konservierung ihrer Antigene untersucht.

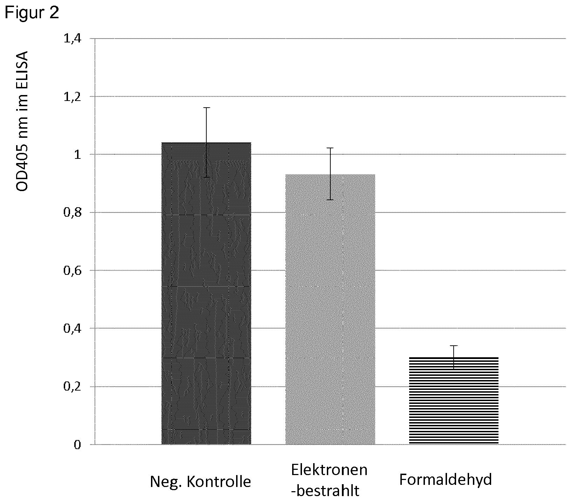

Für die Aktivitätsbestimmung wurden die Viren (und die unbehandelten Kontrollen) auf Marc145 Zellen gegeben und der TCID50-Wert drei Tage später ermittelt. Durch die Bestrahlung ergab sich gegenüber der Kontrolle eine Reduktion der Aktivität um 2,5 Log-Stufen (Figur 1). Die Qualität der Antigene wurde über Messungen mit Antikörpern untersucht. Dazu wurden Seren von Schweinen untersucht, die mit einem Impfstamm von PRRSV infiziert worden waren. Die Immunisierung mit einem lebenden Virus führt zu einer umfassenden humoralen Immunantwort gegen die Antigene in ihrem unbeschädigten Originalzustand. Daher ist das Ausmaß der Bindung von polyklonalen Antikörpern aus einem so immunisierten Tier an ein Antigen ein direkter Indikator für die Unversehrtheit des Antigens. Figur 2 zeigt deutlich, dass sich die Bindungseigenschaften des Schweineserums an die PRRS-Viren durch die Bestrahlung der Viren kaum geändert haben. Folglich befinden sich so gut wie alle Antigene noch in ihrem unbeschädigten Originalzustand, obwohl das Virus um 2,5 Log-Stufen inaktiviert wurde. Zum Vergleich wurden PRRS-Viren auch mit Formaldehyd inaktiviert. Dieser Prozess führte zu einer deutlichen Verminderung des ELISA-Signals, und daher zu einer klaren Zerstörung der Antigene.

Außerdem wurde untersucht, inwieweit der Inaktivierungsprozess die Virus-Struktur beeinträchtigt. Dies wurde mit einem Antikörper gegen das Capsid-Protein (N-protein) des PRRSV gemessen. Dieses Protein wird durch die intakte Virushülle geschützt und ist nicht zugänglich für Antikörper. Ein Signal weist deshalb auf eine beschädigte Virushülle hin. Wie Figur 3 zeigt bleibt die Virushülle nach der Bestrahlung intakt, währen es bei der Peroxid-Aktivierung nach (Amanna et al., supra) zu einer deutlichen Beeinträchtigung der Struktur kommt. Diese Daten weisen darauf hin, dass bei der Elektronenbestrahlung die Inaktivierung vermutlich vor allem auf der Zerstörung der Nukleinsäuren beruht, während die Antigene und die Virusstruktur weitgehend unverändert bleiben.

Daher eignet sich die beschriebene Methode zur Herstellung von inaktivierten Virus-Partikeln, z.B. für die Herstellung von Impfstoffen, wo sie klare Vorteile gegenüber Formaldehyd hat: die Antigene werden weitaus besser konserviert, und es kann auf den Zusatz giftiger Chemikalien verzichtet werden.

### Beispiel 2: Materialien und Methoden

### Viruskultur, Inaktivierung und Bestrahlung

Zellkulturen von Marc145-zellen wurden mit PRRSV (Impfstamm DV) infiziert. Nach drei Tagen wurden die Überstände abgenommen und bei 4°C für 15 Minuten bei 4.000 x g zentrifugiert. Die so geklärten Überstände wurden auf ein 15% Sucrose-Kissen geschichtet und für drei Stunden bei 100.000 x g ultrazentrifugiert. Der Überstand wurde abgenommen, und das Pellet in sterilem PBS (*phosphate buffered saline*, pH 7,4) resuspendiert. Nach Bestimmung der Infektiosität wurde die Virus-Suspension auf eine Konzentration von 2*10⁵ TCID50 /mL eingestellt. Jeweils 100 µL dieser Lösung wurde in 6-well Platten (welche vorher mit 0,5 % Agarose beschichtet waren) gegeben und bei 50, 100 und 200 kGy bestrahlt. NegativKontrollen wurden bis auf die Bestrahlung gleich behandelt.

Nach der Bestrahlung wurde die Virus-haltige Lösung abgenommen und in TCID50-und Antigen-Messungen weiter verwendet. PRRSV wurde mit 0,3% Formaldehyd für 22 Stunden inaktiviert. Danach wurde das Formaldehyd über Dialyse wieder aus der Virus-Suspension entfernt. Die Inaktivierung über Peroxid erfolgte nach Amanna et al. (supra) in 3% H₂O₂ für 22 Stunden, mit anschließender Dialyse.

### TCID50-Messungen

Um die Aktivität der bestrahlten Viren zu untersuchen, wurden Verdünnungsreihen (jeweils 1:10-Schritte) der Virus-Suspensionen auf Marc145-Zellen in 96-well Platten gegeben. Drei Tage später wurde der zytopathische Effekt (CPE) bestimmt. Der TCID50 entspricht die Verdünnung, bei der 50% der infizierten Zellkultur-wells noch einen CPE aufweisen.

### Antigen-Messungen

1,5 µL der Virus-Suspension (bestrahlte und Kontroll-Proben) wurden über Nacht bei 4°C auf 96-well Mikrotiter-Platten inkubiert. Am nächsten Tag wurde ein ELISA (*enzyme linked immunosorbent assay*) nach standard-Protokoll durchgeführt. Zur Detektion der Antigene wurde ein Serum eines PRRSV (Impfstamm DV)-infizierten Schweines verwendet (Verdünnung 1:100). Zur Detektion wurde ein sekundärer anti-Schwein-IgG Antikörper verwendet (konjugiert mit *horseradish peroxidase*, Zymed), in Verdünnung 1:5000.

### Bestrahlung mit Elektronen

Die Zusammensetzung enthaltend PRRSV Viren wurde für die Bestrahlung dünn auf eine große Agarosefläche aufgebracht. Im Detail wurde folgendes durchgeführt:
1.) Herstellung von 0,5% Agarosegelen in PBS,
2.) Gießen der Gele in Gelgießapparatur mit 1 mm Schichtdicke,
3.) Ausstechen der Gele auf kreisrunde Form mit 3,5 cm Durchmesser,
4.) Trocknen der Gele (ca. 14 h unter laufender Sterilwerkbank) in Petrischalen (3,5 cm Durchmesser), die Dosimetrie-Negativkontrollen wurden dann bereits mit eingebrachter Dosimeterfolie getrocknet,
5.) Aufgeben von 100 µL Virensuspension (reines PBS für Dosimetrie-Negativkontrollen, ca. 15 min. Einwirkzeit),
6.) Verpackung mit PET/PE-Folie,
7.) Bestrahlung unter den Bedingungen wie unten angegeben.

Die Bestrahlung erfolgte durch quasistationäre Bestrahlung von jeweils 100 ml Medium an Luft.

Es wurde ein kontinuierlich arbeitender Elektronen-Bandstrahler (Typ Navarone Hersteller: COMET) verwendet. Die Elektronen wurden auf 150 keV beschleunigt, der Strahlstrom war 5 mA. Distanz Luft zwischen Zusammensetzung enthaltend PRRSV Viren und dem Elektronenaustrittsfenster: 45 mm.

Die Applikation der Energiedosen von 50, 100 und 200 kGy erfolgte in Einzelschritten zu je 25 kGy (entsprechend 2, 4, oder 8 Durchläufe der Proben bzw. lineare Durchfahrten mit je 115 mm/sek). Die Zieldosen konnten unter NormaldruckAtmosphäre Luft mit einer Genauigkeit von ca. ± 10 % erreicht werden.

Die Dokumentation der applizierten Dosis erfolgte spektrometrisch mittels Pararosanilin-Cyanid-Dosimeterfolie und RisöScan-System bei einer Messwellenlänge von 554 nm. Für die Bestrahlung mit 100 kGy wurde die Dosimeterfolie nach 50 kGy gewechselt, da die genannte Dosimeterart einen Messbereich bis maximal 80 kGy aufweist. Für die Zieldosis von 200 kGy wurde entsprechend nach 50, 100 und 150 kGy die Dosimeterfolie gewechselt.

Die Nullprobe zeigte keinen Dosiseintrag, d.h. die applizierte Dosis ist ausschließlich durch die Elektronenstrahlbehandlung zurück zu führen und der Kontakt mit PBS sowie den Agarosegelen verursachte keine Veränderung der Dosimeterstreifen.

### Beispiel 3: Bestrahlung von Influenza-Viren

Influenza A Viren (*equine* Influenza H3N8, Stamm A/Equine 2/ Miami/1/63) wurden auf MDCK Zellen vermehrt, und analog zu PRRS-Viren (Beispiele 1 und 2) über Ultrazentrifugation aufkonzentriert.

Die Bestrahlung mit Elektronenstrahlen erfolgte ebenfalls analog zu den Beispielen 1 und 2, jedoch mit einer Dosis von 0 kGy (Kontrolle) und 200 kGy.

Die Aktivitätsmessungen erfolgten über eine TCID50 Endpunktbestimmung. Die Antigene wurden mit dem Serum eines Influenza A-infizierten Menschen im ELISA-Format untersucht.

Wie bei PRRSV zeigt sich eine Inaktivierung der Influenza-Viren. Bei einer Dosis von 200 kGy waren keine aktiven Viren in der Zellkultur mehr nachweisbar (Figur 4). Trotzdem sind die Antigene noch vorhanden und weitgehend unverändert (Figur 5).

## Patentansprüche

1. Verfahren zur Inaktivierung von Viren, **dadurch gekennzeichnet,**
**dass** eine immunogene Zusammensetzung oder Vakzine enthaltend mindestens ein Virus mit Elektronenstrahlen bestrahlt wird,
wobei
die immunogene Zusammensetzung oder Vakzine enthaltend mindestens ein Virus mit einer Elektronenstrahldosis im Bereich von 15 bis 110 kGy bestrahlt wird und die Bestrahlungszeit im Bereich von zwischen 1 Sekunde und 100 Sekunden ist, und die Elektronenstrahlen mit einer Beschleunigungsenergie von zwischen 150 keV und 700 keV beschleunigt werden,
und die immunogene Zusammensetzung oder Vakzine enthaltend mindestens ein Virus
(i) flüssig ist, insbesondere eine Suspension ist, und
(ii) mindestens ein virales Immunogen enthält.

2. Verfahren nach Anspruch 1, **dadurch gekennzeichnet,**
A) **dass** das mindestens ein Virus ausgewählt ist aus:
(i) einem behüllten Virus oder unbehüllten Virus, und/oder
(ii) einem dsDNA Virus, dsRNA Virus, ssRNA Virus, oder ssDNA Virus, und/oder
(iii) einem humanpathogenen und/oder tierpathogenen Virus, bevorzugt, dass das mindestens ein Virus ausgewählt ist aus einem humanpathogenen und/oder tierpathogenen behüllten dsRNA Virus, behüllten ss(-)RNA Virus, oder behüllten ss(+)RNA Virus,
besonders bevorzugt
a) **dass** das mindestens ein Virus ausgewählt ist aus einem humanpathogenen und/oder tierpathogenen ss(+)RNA-Virus, ganz besonders bevorzugt, dass das mindestens ein Virus ausgewählt ist aus einem Virus der *Arteriviridae* Familie, noch mehr bevorzugt, dass das mindestens ein Virus ein *Porcine reproductive and respiratory syndrome virus* (PRRS Virus) ist,
oder
b) **dass** das mindestens ein Virus ausgewählt ist aus einem tierpathogenen Virus, dem Influenza A und B Virus, dem FSME Virus, dem IPV Virus und dem Hepatitis A Virus,
und/oder
B) **dass** die Vakzine oder immunogene Zusammensetzung (i) ein Virus oder (ii) zwei oder mehr unterschiedliche Viren enthält.

3. Verfahren nach einem der Ansprüche 1 bis 2, **dadurch gekennzeichnet,**
A) **dass** die Elektronenstrahlen mit einer Beschleunigungsenergie von zwischen 200 keV und 500 keV beschleunigt werden, bevorzugt mit einer Beschleunigungsenergie von zwischen 250 keV und 400 keV beschleunigt werden, und/oder die Elektronenstrahlen im Wesentlichen unter Normaldruckatmosphäre appliziert werden, bevorzugt wobei die Normaldruckatmosphäre als Luftsauerstoff, Stickstoff oder Kohlendioxidgas vorliegt, und/oder
B) **dass** die immunogene Zusammensetzung oder Vakzine enthaltend mindestens ein Virus mit einer Elektronenstrahldosis im Bereich von 50 kGy bis 110 kGy bestrahlt wird, und/oder
C) **dass** die Aktivität des mindestens einen Virus, bevorzugt gemessen als TCID50-Wert, nach Bestrahlung weniger als 5%, bevorzugt weniger als 1%, mehr bevorzugt weniger als 0,1% der Aktivität vor der Bestrahlung ist, noch mehr bevorzugt, dass nach Bestrahlung keine Aktivität des mindestens einen Virus mehr nachweisbar ist.

4. Verfahren nach einem der Ansprüche 1 bis 3, **dadurch gekennzeichnet,**
A) **dass** die Antigenstruktur der Viren der immunogenen Zusammensetzung oder Vakzine nach Bestrahlung im Wesentlichen erhalten bleibt,
bevorzugt, dass die Bindung eines polyklonalen Serums gerichtet gegen das nicht-inaktivierte Virus an das mindestens ein Virus der bestrahlten immunogenen Zusammensetzung oder Vakzine mindestens 40%, bevorzugt mindestens 70%, mehr bevorzugt mindestens 80%, noch mehr bevorzugt mindestens 90% der Bindung des polyklonalen Serums an das mindestens ein Virus der immunogenen Zusammensetzung oder Vakzine vor der Bestrahlung ist,
insbesondere wobei die Bindung des polyklonalen Serums an das mindestens ein Virus der immunogenen Zusammensetzung oder Vakzine mittels ELISA bestimmt wird,
und/oder
B) **dass** die Virusstruktur der Viren nach Bestrahlung im Wesentlichen erhalten bleibt.

5. Verfahren nach einem der Ansprüche 1 bis 4, **dadurch gekennzeichnet, dass**
(a) die Bestrahlung unter Verwendung einer Vorrichtung zur Erzeugung von Elektronenstrahlen durchgeführt wird,
(i) die kontinuierlich arbeitet, und/oder
(ii) die nach dem Kalt- oder Glühkathodenprinzip Elektronen liefert und/oder
(iii) die als Axialstrahler (Scanner) oder Linear-Breitbandstrahler ausgeführt ist,
und/oder
(iv) die Elektronen nach Austritt aus der evakuierten Erzeugerkammer der Vorrichtung durch ein Austrittsfenster auf die immunogene Zusammensetzung oder Vakzine appliziert werden, wobei die immunogene Zusammensetzung oder Vakzine bevorzugt in einem Container ist, und/oder
(v) die immunogene Zusammensetzung oder Vakzine statisch in die Vorrichtung aufgenommen wird, oder kontinuierlich durch den Elektronenstrahl transportiert wird,
und/oder
(b) die Dosisrate im Bereich von 1 kGy/0,1 sek bis 150 kGy/1000 sek liegt, und/oder
(c) die Temperatur der immunogenen Zusammensetzung oder Vakzine vor der Bestrahlung zwischen 1°C und 40°C, vorzugsweise zwischen 5°C und 37°C, stärker bevorzugt zwischen 10°C und 32°C, noch stärker bevorzugt zwischen 15°C und 30°C liegt, und/oder
(d) die Temperaturerhöhung der immunogenen Zusammensetzung oder Vakzine nach der Bestrahlung im Vergleich zu vor der Bestrahlung zwischen 1°C und 15°C, vorzugsweise zwischen 2°C und 10°C liegt, und/oder
(e) die Temperatur der immunogenen Zusammensetzung oder Vakzine nach der Bestrahlung zwischen 2°C und 41°C, vorzugsweise zwischen 6°C und 38°C, stärker bevorzugt zwischen 11°C und 33°C, noch stärker bevorzugt zwischen 16°C und 31°C liegt, und/oder
(f) die Dichte der immunogenen Zusammensetzung oder Vakzine zwischen 0,9 und 2 g/cm³, bevorzugt zwischen 1,0 und 1,8 g/cm³ liegt, und/oder
(g) die immunogene Zusammensetzung oder Vakzine enthaltend mindestens ein Virus eine flüssige Suspension enthaltend Wasser ist, bevorzugt eine Suspension des mindestens ein Virus in einer wässrigen Lösung ist, wobei die wässrige Lösung besonders bevorzugt eine oder mehrere Puffersubstanzen enthält und/oder
(h) die immunogene Zusammensetzung oder Vakzine
(a) einen oder mehrere Adjuvantien, und/oder
(b) pharmazeutisch annehmbare Träger- und/oder Hilfsstoffe, und/oder
(c) ein oder mehrere weitere Immunogene enthält,
vorzugsweise wobei das eine oder mehrere weitere Immunogen ausgewählt ist aus:
(i) einer organischen Substanz, insbesondere einem Protein, das glykosyliert oder unglykosyliert sein kann, einer Nukleinsäure, einem Toxin, oder einem Zuckermolekül, das optional an einen Träger gebunden ist, und
(ii) einem Virus oder einem Lebewesen, insbesondere einem Bakterium, wobei das Virus oder Lebewesen aktiv oder inaktiviert sein kann.

6. Verfahren zur Herstellung einer Vakzine, enthaltend mindestens ein virales Immunogen, insbesondere einer Vakzine umfassend einen viralen Ganzpartikelimpfstoff, **dadurch gekennzeichnet, dass**:
(a) das Verfahren nach einem der Ansprüche 1 bis 5 durchgeführt wird,
(b1) der immunogenen Zusammensetzung enthaltend mindestens ein Virus optional ein oder mehrere Adjuvantien zugegeben werden, und/oder
(b2) der immunogenen Zusammensetzung enthaltend mindestens ein Virus optional ein oder mehrere pharmazeutisch annehmbare Träger- und/oder Hilfsstoffe zugegeben werden, und/oder
(b3) der immunogenen Zusammensetzung enthaltend mindestens ein Virus optional ein oder mehrere weitere Immunogene zugegeben werden,
wobei die Schritte (a) bis (b3) in beliebiger Reihenfolge durchgeführt werden, vorzugsweise wobei folgende weitere Schritte durchgeführt werden:
(c) Sterilisieren der immunogenen Zusammensetzung, und/oder
(d) Abfüllen der immunogenen Zusammensetzung in einen Container, wobei die Schritte (a) bis (d) in beliebiger Reihenfolge durchgeführt werden können,
und anschließend an die Schritte (a) bis (d) die Vakzine optional getrocknet, gefriergetrocknet oder eingefroren wird.

7. Verwendung von Elektronenstrahlen
(a) zur Inaktivierung von Viren in einer immunogenen Zusammensetzung oder Vakzine enthaltend mindestens ein Virus, wobei die immunogene Zusammensetzung oder Vakzine
(i) flüssig ist, insbesondere eine Suspension ist, und
(ii) mindestens ein virales Immunogen enthält,
und/oder
(b) zur Herstellung eines inaktivierten viralen Ganzpartikelimpfstoffs,
wobei die Elektronenstrahldosis im Bereich von 15 bis 110 kGy und die Bestrahlungszeit im Bereich von zwischen 1 Sekunde und 100 Sekunden ist, und die Elektronenstrahlen mit einer Beschleunigungsenergie von zwischen 150 keV und 700 keV beschleunigt werden.

8. Verwendung nach Anspruch 7, wobei die Elektronenstrahlen mit einer Beschleunigungsenergie von zwischen 200 keV und 500 keV beschleunigt werden, bevorzugt mit einer Beschleunigungsenergie von zwischen 250 keV und 400 keV beschleunigt werden, und/oder die Elektronenstrahlen im Wesentlichen unter Normaldruckatmosphäre appliziert werden, bevorzugt wobei die Normaldruckatmosphäre als Luftsauerstoff, Stickstoff oder Kohlendioxidgas vorliegt.

9. Verwendung nach Anspruch 7 oder 8, wobei die Elektronenstrahldosis im Bereich von 50 kGy bis 110 kGy liegt.

10. Verwendung eines Verfahrens nach einem der Ansprüche 1 bis 6 zur Herstellung eines inaktivierten viralen Ganzpartikelimpfstoffs.

## Claims

1. Method for inactivating viruses, **characterized in that** an immunogenic composition or vaccine containing at least one virus is irradiated with electron beams wherein
the immunogenic composition or vaccine containing at least one virus is irradiated with an electron beam dose in the range from 15 to 110 kGy and the irradiation time is in the range from between 1 second and 100 seconds, and the electron beams are accelerated with an acceleration energy of between 150 keV and 700 keV,
and the immunogenic composition or vaccine containing at least one virus
(i) is liquid, in particular is a suspension, and
(ii) contains at least one viral immunogen.

2. Method according to Claim 1, **characterized in that**
A) the at least one virus is selected from:
(i) an enveloped virus or naked virus, and/or
(ii) a dsDNA virus, dsRNA virus, ssRNA virus, or ssDNA virus, and/or
(iii) a virus pathogenic in humans and/or animals,
preferably that the at least one virus is selected from a enveloped dsRNA virus, enveloped ss(-)RNA virus or enveloped ss(+)RNA virus, pathogenic in humans and/or animals
particularly preferably
a) that the at least one virus is selected from a ss(+)RNA virus pathogenic in humans and/or animals, quite especially preferably that the at least one virus is selected from a virus of the *Arteriviridae* family, still more preferably, that the at least one virus is a *Porcine reproductive and respiratory syndrome virus* (PRRS virus),
or
b) that the at least one virus is selected from a virus pathogenic in animals, the influenza A and B virus, the FSME virus, the IPV virus and the hepatitis A virus.
and/or
B) that the vaccine or immunogenic composition contains (i) one virus or (ii) two or more different viruses.

3. Method according to one of Claims 1 to 2, **characterized in that**
A) the electron beams are accelerated with an acceleration energy of between 200 keV and 500 keV, preferably with an acceleration energy of between 250 keV and 400 keV, and/or the electron beams are applied essentially under a normal atmospheric pressure, preferably wherein the normal pressure atmosphere is present as atmospheric oxygen, nitrogen or carbon dioxide gas, and/or
B) that the immunogenic composition or vaccine containing at least one virus is irradiated with an electron beam dose in the range from 50 kGy to 110 kGy, and/or
C) that the activity of the at least one virus, preferably measured as TCID-50 value, after irradiation is less than 5%, preferably less than 1%, more preferably less than 0.1% of the activity before the irradiation, still more preferably that after irradiation activity of the at least one virus is no longer detectable.

4. Method according to one of Claims 1 to 3, **characterized in that**
A) the antigen structure of the viruses of the immunogenic composition or vaccine is essentially retained after irradiation,
preferably that the binding of a polyclonal serum directed against the non-inactivated virus onto the at least one virus of the irradiated immunogenic composition or vaccine is at least 40%, preferably at least 70%, more preferably at least 80%, still more preferably at least 90% of the binding of the polyclonal serum onto the at least one virus of the immunogenic composition or vaccine before the irradiation,
in particular wherein the binding of the polyclonal serum onto the at least one virus of the immunogenic composition or vaccine is determined by ELISA,
and/or
B) that the virus structure of the viruses is essentially retained after irradiation.

5. Method according to one of Claims 1 to 4, **characterized in that**
(a) the irradiation is performed with use of a device for generating electron beams,
(i) which operates continuously, and/or
(ii) which supplies electrons according to the cold or hot cathode principle and/or
(iii) which is implemented as an axial emitter (scanner) or linear broadband emitter, and/or
(iv) the electrons after exiting from the evacuated generator chamber of the device through an exit window are applied onto the immunogenic composition or vaccine, wherein the immunogenic composition or vaccine is preferably in a container, and/or
(v) the immunogenic composition or vaccine is taken up into the device statically, or is continuously transported through the electron beam, and/or
(b) the dose rate lies in the range from 1 kGy/0.1 sec to 150 kGy/1000 sec, and/or
(c) the temperature of the immunogenic composition or vaccine before the irradiation lies between 1°C and 40°C, preferably between 5°C and 37°C, more strongly preferably between 10°C and 32°C, still more strongly preferably between 15°C and 30°C, and/or
(d) the temperature increase of the immunogenic composition or vaccine after the irradiation in comparison to before the irradiation lies between 1°C and 15°C, preferably between 2°C and 10°C, and/or
(e) the temperature of the immunogenic composition or vaccine after the irradiation lies between 2°C and 41°C, preferably between 6°C and 38°C, more strongly preferably between 11°C and 33°C, still more strongly preferably between 16°C and 31°C, and/or
(f) the density of the immunogenic composition or vaccine lies between 0.9 and 2 g/cm³, preferably between 1.0 and 1.8 g/cm³, and/or
(g) the immunogenic composition or vaccine containing at least one virus is a liquid suspension containing water, preferably a suspension of the at least one virus in an aqueous solution, wherein the aqueous solution particularly preferably contains one or more buffer substances and/or
(h) the immunogenic composition or vaccine contains
(a) one or more adjuvants, and/or
(b) pharmaceutically acceptable carrier and/or auxiliary substances, and/or
(c) one or more further immunogens,
preferably wherein the one or more further immunogen is selected from:
(i) an organic substance, in particular a protein, which can be glycosylated or unglycosylated, a nucleic acid, a toxin, or a sugar molecule, which is optionally bound onto a carrier, and
(ii) a virus or an organism, in particular a bacterium, wherein the virus or organism can be active or inactivated.

6. Method for production of a vaccine, containing at least one viral immunogen, in particular a vaccine comprising a viral whole particle vaccine, **characterized in that**:
(a) the method according to one of Claims 1 to 5 is performed,
(b1) optionally one or more adjuvants are added to the immunogenic composition containing at least one virus, and/or
(b2) optionally one or more pharmaceutically acceptable carrier and/or auxiliary substances are added to the immunogenic composition containing at least one virus, and/or
(b3) optionally one or more further immunogens are added to the immunogenic composition containing at least one virus,
wherein the steps (a) to (b3) are performed in any order, preferably wherein the following further steps are performed:
(c) sterilization of the immunogenic composition, and/or
(d) filling of the immunogenic composition into a container,
wherein the steps (a) to (d) can be performed in any order,
and following the steps (a) to (d) the vaccine is optionally dried, freeze-dried or frozen.

7. Use of electron beams
(a) for the inactivation of viruses in an immunogenic composition or vaccine containing at least one virus, wherein the immunogenic composition or vaccine
(i) is liquid, in particular a suspension, and
(ii) contains at least one viral immunogen,
and/or
(b) for the production of an inactivated viral whole particle vaccine.
wherein the electron beam dose is in the range from 15 to 110 kGy and the irradiation time is in the range of between 1 second and 100 seconds, and the electron beams are accelerated with an acceleration energy of between 150 keV and 700 keV.

8. Use according to Claim 7, wherein the electron beams are accelerated with an acceleration energy of between 200 keV and 500 keV, preferably with an acceleration energy of between 250 keV and 400 keV, and/or the electron beams are applied essentially under a normal pressure atmosphere, preferably wherein the normal pressure atmosphere is present as atmospheric oxygen, nitrogen or carbon dioxide.

9. Use according to Claim 7 or 8, wherein the electron beam dose lies in the range from 50 kGy to 110 kGy.

10. Use of a method according to one of Claims 1 to 6 for the production of an inactivated viral whole particle vaccine.

## Revendications

1. Procédé permettant l'inactivation de virus, **caractérisé en ce qu'**une composition immunogène ou un vaccin contenant au moins un virus est irradié(e) avec des faisceaux électroniques,
dans lequel
la composition immunogène ou le vaccin contenant au moins un virus est irradié (e) avec une dose de faisceaux électroniques dans la plage de 15 à 110 kGy, et le temps d'irradiation est dans la plage comprise entre 1 seconde et 100 secondes, et les faisceaux électroniques sont accélérés avec une énergie d'accélération comprise entre 150 keV et 700 keV,
et la composition immunogène ou le vaccin contenant au moins un virus
(i) est liquide, est notamment une suspension, et
(ii) contient au moins un immunogène viral.

2. Procédé selon la revendication 1, **caractérisé en ce que**
A) l'au moins un virus est choisi parmi :
(i) un virus encapsidé ou un virus non encapsidé, et/ou
(ii) un virus d'ADNdb, un virus d'ARNdb, un virus d'ARNsb ou un virus d'ADNsb, et/ou
(iii) un virus pathogène pour les humains et/ou pathogène pour les animaux,
de préférence **en ce que** l'au moins un virus est choisi parmi un virus d'ARNdb encapsidé, un virus d'ARN(-)sb encapsidé ou un virus d'ARN(+)sb encapsidé, pathogènes pour les humains et/ou pathogènes pour les animaux, de manière particulièrement préférée
a) que l'au moins un virus est choisi parmi un virus d'ARN(+)sb, pathogène pour les humains et/ou pathogène pour les animaux, de manière toute particulièrement préférée **en ce que** l'au moins un virus est choisi parmi un virus de la famille des *Arteriviridae,* de manière encore mieux préférée que l'au moins un virus soit un *virus du syndrome dysgénésique et respiratoire du porc* (virus PRRS),
ou
b) **en ce que** l'au moins un virus est choisi parmi un virus pathogène pour les animaux, le virus de la grippe A et B, le virus FSME, le virus IPV et le virus de l'hépatite A,
et/ou
B) le vaccin ou la composition immunogène contient (i) un virus ou (ii) deux ou plus de deux virus différents.

3. Procédé selon l'une des revendications 1 à 2, **caractérisé en ce que**
A) les faisceaux électroniques sont accélérés avec une énergie d'accélération comprise entre 200 keV et 500 keV, de préférence avec une énergie d'accélération comprise entre 250 keV et 400 keV, et/ou les faisceaux électroniques sont appliqués sensiblement sous pression atmosphérique normale, la pression atmosphérique normale existant de préférence en tant qu'oxygène de l'air, azote ou dioxyde de carbone gazeux, et/ou
B) la composition immunogène ou le vaccin contenant au moins un virus est irradié (e) avec une dose de faisceaux électroniques dans la plage comprise de 50 kGy à 110 kGy, et/ou
C) l'activité dudit au moins un virus, de préférence mesurée en tant que valeur TCID₅₀, après irradiation est inférieure à 5 %, de préférence inférieure à 1 %, plus préférablement inférieure à 0,1 % de l'activité avant l'irradiation, encore plus préférablement **en ce qu'**aucune activité dudit au moins un virus n'est plus détectable après l'irradiation.

4. Procédé selon l'une des revendications 1 à 3, **caractérisé en ce que**
A) la structure d'antigène des virus de la composition immunogène ou du vaccin reste sensiblement intacte après irradiation,
de préférence que la liaison d'un sérum polyclonal dirigé contre le virus non inactivé audit au moins un virus de la composition immunogène ou du vaccin irradié(e) est d'au moins 40 %, de préférence d'au moins 70 %, plus préférablement d'au moins 80 %, encore plus préférablement d'au moins 90 % de la liaison du sérum polyclonal audit au moins un virus de la composition immunogène ou du vaccin avant l'irradiation,
notamment où la liaison du sérum polyclonal audit au moins un virus de la composition immunogène ou du vaccin avant l'irradiation est déterminée par ELISA,
et/ou
B) la structure virale des virus reste sensiblement intacte après irradiation.

5. Procédé selon l'une des revendications 1 à 4, **caractérisé en ce que**
(a) l'irradiation est réalisée en utilisant un dispositif pour la génération de faisceaux électroniques,
(i) qui travaille en continu, et/ou
(ii) qui émet des électrons d'après le principe des cathodes froides ou chaudes et/ou
(iii) qui est exécuté sous forme d'émetteur de faisceaux axiaux (scanner) ou émetteur de larges bandes linéaires,
et/ou
(iv) les électrons sont appliqués après sortie de la chambre du générateur mise sous vide à travers une fenêtre de sortie sur la composition immunogène ou le vaccin, où la composition immunogène ou le vaccin est de préférence dans un conteneur, et/ou
(v) la composition immunogène ou le vaccin est abrité(e) statiquement dans le dispositif ou transporté(e) en continu par le faisceau électronique,
et/ou
(b) la dose se situe dans la plage de 1 kGy/0,1 sec à 150 kGy/1000 sec, et/ou
(c) la température de la composition immunogène ou du vaccin avant l'irradiation est comprise entre 1°C et 40°C, de préférence entre 5°C et 37°C, le plus préférablement entre 10°C et 32°C, de manière encore plus fortement préférée entre 15°C et 30°C, et/ou
(d) la hausse de température de la composition immunogène ou du vaccin après l'irradiation par rapport à celle avant l'irradiation est comprise entre 1°C et 15°C, de préférence entre 2°C et 10°C, et/ou
(e) la température de la composition immunogène ou du vaccin après irradiation est comprise entre 2°C et 41°C, de préférence entre 6°C et 38°C, le plus préférablement entre 11°C et 33°C, de manière encore plus fortement préférée entre 16°C et 31°C, et/ou
(f) la densité de la composition immunogène ou du vaccin est comprise entre 0,9 et 2 g/cm³, de préférence entre 1,0 et 1,8 g/cm³, et/ou
(g) la composition immunogène ou le vaccin contenant au moins un virus est une suspension liquide contenant de l'eau, de préférence une suspension d'au moins un virus dans une solution aqueuse, où la solution aqueuse contient de préférence une ou plusieurs substances tampons et/ou
(h) la composition immunogène ou le vaccin contient
(a) un ou plusieurs adjuvants, et/ou
(b) des supports et/ou auxiliaires pharmaceutiquement acceptables, et/ou
(c) un ou plusieurs autres immunogènes,
où de préférence lesdits un ou plusieurs autres immunogènes sont choisis parmi :
(i) une substance organique, en particulier une protéine, qui peut être glycosylée ou non glycosylée, un acide nucléique, une toxine ou une molécule de sucre, qui est éventuellement liée à un support, et
(ii) un virus ou un être vivant, en particulier une bactérie, où le virus ou l'être vivant peut être actif ou inactivé.

6. Procédé de préparation d'un vaccin, contenant au moins un immunogène viral, en particulier d'un vaccin comprenant une substance vaccinale virale à particules entières, **caractérisé en ce que**
(a) le procédé selon l'une des caractéristiques 1 à 5 est réalisé,
(b1) éventuellement un ou plusieurs adjuvants sont ajoutés à la composition immunogène contenant au moins un virus, et/ou
(b2) éventuellement un ou plusieurs supports et/ou auxiliaires pharmaceutiquement acceptables sont ajoutés à la composition immunogène contenant au moins un virus, et/ou
(b3) éventuellement un ou plusieurs autres immunogènes sont ajoutés à la composition immunogène contenant au moins un virus,
dans lequel les étapes (a) à (b3) sont réalisées dans un ordre quelconque,
de préférence dans lequel les autres étapes suivantes sont réalisées :
(c) la composition immunogène est stérilisée et/ou
(d) la composition immunogène est déchargée dans un conteneur,
dans lequel les étapes (a) à (d) sont réalisées dans un ordre quelconque,
et consécutivement aux étapes (a) à (d), le vaccin est éventuellement séché, lyophilisé ou congelé.

7. Utilisation de faisceaux électroniques
(a) pour l'inactivation de virus dans une composition immunogène ou un vaccin contenant au moins un virus, où la composition immunogène ou le vaccin
(i) est liquide, est notamment une suspension, et
(ii) contient au moins un immunogène viral, et/ou
(b) pour la préparation d'une substance vaccinale virale inactivée à particules entières,
dans laquelle la dose de faisceaux électroniques est comprise dans la plage de 15 à 110 kGy, et le temps d'irradiation est dans la plage comprise entre 1 seconde et 100 secondes, et les faisceaux électroniques sont accélérés avec une énergie d'accélération comprise entre 150 keV et 700 keV.

8. Utilisation selon la revendication 7, dans laquelle les faisceaux électroniques sont accélérés avec une énergie d'accélération comprise entre 200 keV et 500 keV, de préférence avec une énergie d'accélération comprise entre 250 keV et 400 keV, et/ou les faisceaux électroniques sont appliqués sensiblement sous pression atmosphérique normale, la pression atmosphérique normale existant de préférence en tant qu'oxygène de l'air, azote ou dioxyde de carbone gazeux.

9. Utilisation selon la revendication 7 ou 8, dans laquelle la dose de faisceaux électroniques est comprise dans la plage de 50 kGy à 110 kGy.

10. Utilisation d'un procédé selon l'une des revendications 1 à 6 pour la préparation d'une substance vaccinale virale inactivée à particules entières.
